# EUROPÄISCHE PATENTSCHRIFT

(11) **EP 1 399 408 B1**
(45) Veröffentlichungstag und Bekanntmachung des Hinweises auf die Patenterteilung: **22.03.2006**
(21) Anmeldenummer: 02778851.2
(22) Anmeldetag: 28.05.2002
(51) Int. Cl.: C07C 67/54, C07C 67/03, C07C 69/54

(54) **VERFAHREN ZUR HERSTELLUNG VON (METH)ACRYLSÄUREESTERN**
METHOD FOR PRODUCING (METH)ACRYLIC ACID ESTERS
PROCEDE DE PRODUCTION D'ESTERS D'ACIDE (METH)ACRYLIQUE

(30) Priorität: 08.06.2001 DE 10127939
(43) Veröffentlichungstag der Anmeldung: 24.03.2004
(73) Patentinhaber: BASF Aktiengesellschaft, 67056 Ludwigshafen (DE)
(72) Erfinder: GEISENDÖRFER, Matthias, 67435 Neustadt (DE); DAMS, Albrecht, 67157 Wachenheim (DE); NESTLER, Gerhard, A-1070 Wien (AT)
(86) Internationale Anmeldenummer: PCT/EP2002/005820
(87) Internationale Veröffentlichungsnummer: WO 2002/100814

(56) Entgegenhaltungen:
- EP-A- 0 298 867
- EP-A- 0 902 017
- EP-A- 0 906 902
- EP-A- 0 960 877
- DE-A- 10 026 644
- US-A- 3 686 268
- US-A- 3 872 161
- CIQUINE CIA PETROQU: "WPI WORLD PATENT INFORMATION DERWENT, DERWENT, GB" , WPI WORLD PATENT INFORMATION DERWENT, DERWENT, GB, VOL. 43, NR. 88 XP002088821 & BR 8 701 337 A (CIQUINE CIA PETROQU) 27. September 1988 (1988-09-27)

## Beschreibung

Die vorliegende Erfindung betrifft ein Verfahren zur kontinuierlichen Herstellung von höheren (Meth)acrylsäureestern durch Umesterung eines niederen (Meth)acrylsäureesters mit einem höheren Alkohol.

Die Herstellung von (Meth)acrylsäureestern durch Umesterung in Gegenwart von sauren oder basischen Katalysatoren ist allgemein bekannt. Als Katalysatoren werden vor allem Titanalkoholate vorgeschlagen, deren Alkylgruppen C₁ - C₄ - Alkylreste darstellen, z.B. Tetramethyl-, Tetraethyl-, Tetraisopropyl-, Tetrapropyl, Tetraisobutyl- und Tetrabutyltitanat (siehe z.B. EP-B1 298 867, EP-A2 960 877). Weiterhin werden als Katalysatoren u. a. Titanphenolate (DE-OS 200 86 18), Metallchelatverbindungen von z. B. Hafnium, Titan, Zirkon oder Calcium, Alkali- und Magnesium-alkoholate, organische Zinnverbindungen oder Calcium- und Lithiumverbindungen, beispielsweise -Oxide, -Hydroxyde, -Carbonate oder -Halogenide vorgeschlagen.

Da es sich bei der Umesterung bekanntlich um eine Gleichgewichtsreaktion handelt, muß einer der Ausgangsstoffe im großen Überschuß eingesetzt werden oder/und eines der Reaktionsprodukte aus dem Gleichgewicht entfernt werden, um wirtschaftliche Umsätze zu erzielen. In der Regel wird daher das bei der Umesterung freigesetzte niedere Alkanol (Alkoholkomponente mit dem niedrigeren Siedepunkt) destillativ abgetrennt. Nachteilig ist dabei, daß die freigesetzten Alkanole, üblicherweise Methanol bzw. Ethanol, mit den entsprechenden (Meth)acrylsäureestern (Methyl- bzw. Ethyl(meth)acrylat) ein Azeotrop bilden und somit nicht direkt destillativ trennbar sind.

Aus ökologischen und ökonomischen Gründen ist die Wiederverwendung des Azeotrops bzw. seiner einzelnen Komponenten (Alkanol und (Meth)acrylester) jedoch vorteilhaft.

Die direkte Verwertung des Azeotropes oder die Wiedergewinnung und Rückführung des Ausgangsesters kann durch das Einschleppen von Verunreinigungen, z.B. durch den Katalysator, erschwert oder sogar verhindert werden.

Durch Verwendung von Titantetraisopropylat oder -butylat, den üblichsten Umesterungskatalysatoren, werden beispielsweise Isopropanol bzw. Butanol als Verunreinigung eingeschleppt (DE-OS 20 08 618, Seite 2).

Werden Titan, Zirkon oder Hafnium-Acetylacetonate als Katalysatoren eingesetzt, findet sich beispielsweise Acetylaceton im Reaktionsgemisch.

Die Verunreinigungen sind wegen des geringen Siedepunktsunterschiedes oder Azeotropbildung z.T. schwer zu entfernen und führen z.T. zur Bildung von weiteren Nebenprodukten (Umesterungsreaktionen, Addition an die Doppelbindung der Ester).

DE-OS 20 08 618 empfiehlt daher die Verwendung von Titanphenolaten als Katalysatoren. Diese müssen aber sehr umständlich und aufwendig hergestellt werden und sind als Feststoffe außerdem schlecht handhabbar.

Prinzipiell könnten derartige Verunreinigungen auch dadurch vermieden werden, daß man Titanate der niederen Alkanole R¹OH (siehe Gleichung I) einsetzt, deren Alkyl(meth)acrylate verwendet werden (z.B. Titantetramethylat bei Verwendung von (Meth)acrylsäuremethylester). EP-B1 298 867 beschreibt beispielsweise die Umesterung von Ethylacrylat mit Dimethylaminoethanol in Gegenwart von Titantetraethanolat. Diese Titanate sind teuer und äußerst hydrolyseempfindlich (DE-OS 20 08 618, Seite 2) und haben daher keine technische Bedeutung.

Weiterhin ist bekannt, daß das bei der Herstellung von basischen (Meth)acrylaten durch Umesterung anfallende Gemisch beziehungsweise Azeotrop aus Ausgangsester und der Alkanolkomponente des Ausgangsesters durch basische Verbindungen (z.B. Amine) verunreinigt ist (EP-A2 906 902). Das Gemisch kann demnach nur dann problemlos bei der Herstellung des Ausgangsesters verwertet werden, wenn es vorher gereinigt wird. EP-A2 906 902 schlägt eine aufwendige Behandlung mit sauren Ionenaustauscherharzen vor.

Nachteilig ist dabei vor allem, daß der Ionenaustauscher umweltbelastend regeneriert (Anfall von Abwasser) und/oder entsorgt werden muß.

Da die Bildung der Azeotrope bzw. Gemische aus Ausgangsester und Ausgangsalkanol demnach einen großen Nachteil darstellt, wurden verschiedene Verfahren zur Vermeidung und/oder Aufarbeitung dieser Azeotrope beziehungsweise Gemische aus Alkanol und (Meth)acrylester vorgeschlagen.

In der Literatur, z.B. in EP-B1 210 907, wird die Verwendung von Hilfsstoffen, wie Benzol, Hexan, Cyclohexan etc. vorgeschlagen, die mit den freiwerdenden niederen Alkanolen Heteroazeotrope bilden. Das bei der Umesterung freigesetzte niedere Alkanol wird dabei mit dem Hilfsstoff als Azeotrop destillativ abgetrennt, wobei das Kondensat in zwei Phasen zerfällt. Die Phase, die den Hilfsstoff enthält wird in die Umesterung zurückgefahren und die mit Hilfsstoff gesättigte Alkanolphase ausgeschleust. Da ein Hilfsstoff benötigt wird und die ausgeschleuste Alkanolphase vor der Wiederverwendung gereinigt werden muß, haben diese Verfahren jedoch keine wirtschaftliche Bedeutung.

Außerdem wurden verschiedene Verfahren zur Trennung/Aufarbeitung dieser Alkanol-Ester-Azeotrope bzw. Gemische vorgeschlagen.

EP-A1 736 510 schlägt vor, die Trennung des Gemisches aus Methanol und (Meth)acrylsäuremethylester sowie gegebenenfalls Wasser mit Hilfe von aliphatischen Kohlenwasserstoffen, die mit Methanol ein Azeotrop bilden, durchzuführen. Der Einsatz eines zusätzlichen Hilfsstoffes macht jedoch auch dieses Verfahren unwirtschaftlich.

DE-A1 23 17 226 schlägt vor, das aus Alkanol und dem entsprechenden (Meth)acrylsäureester gebildete Azeotrop durch Behandlung mit Wasser über Auswaschen des Alkanols zu trennen. Das Verfahren ist nicht wirtschaftlich, da eine wäßrige Alkanollösung anfällt, die entsorgt bzw. aufgearbeitet werden muß, und die Esterphase vor der Rückführung in die Umesterung getrocknet werden muß.

EP-A2 143 639 empfiehlt die Trennung dieser Azeotrope mit komplexbildenden Salzen, z. B. LiCl, und einem Extraktionsmittel. Das Verfahren ist unwirtschaftlich, da es Abwässer produziert und mehrere Destillationsschritte benötigt.

Prinzipiell kann eine Azeotropbildung auch dadurch verhindert werden, daß die Umesterung des niederen Esters mit mindestens stöchiometrischen Mengen eines Metallalkoholats des höheren Alkanols durchgeführt wird. Das dabei gebildete Metallalkoholat des niederen Alkanols wird isoliert und durch Umsetzung mit dem höheren Alkanol wieder in das entsprechende Metallalkoholat umgewandelt, das in die Umesterung zurückgeführt wird (EP-A1 118 639, EP-A2 160 427). Das Verfahren benötigt jedoch große Metallalkoholatmengen und hat daher keine industrielle Bedeutung.

Ein weiteres Problem stellt die Bildung von Michael-Additionsprodukten bei der Umesterung und Aufarbeitung des Umesterungsreaktionsgemisches dar. Unter Michael-Additionsprodukten werden hier die durch Addition von Alkoholen an die Doppelbindung der (Meth)acrylsäureester entstandenen Verbindungen verstanden (EP-A2 906 902, Seiten 8 bis 9).

Es ist allgemein bekannt, daß diese Addition (siehe Gleichung II) besonders in Anwesenheit von alkalischen Katalysatoren erfolgt (Organikum, 17. Auflage, Seite 506, VEB Deutscher Verlag der Wissenschaften, Berlin 1988).

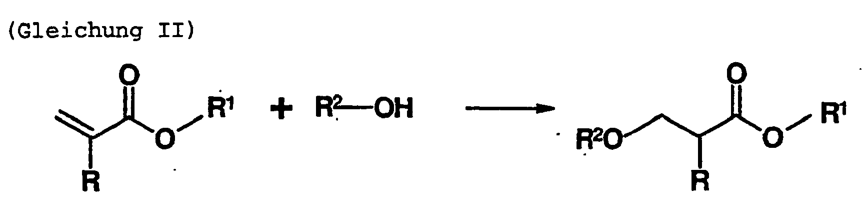

Bei der Umesterung entsprechend Gleichung I spielen im wesentlichen die Addukte (II) und (III) eine Rolle

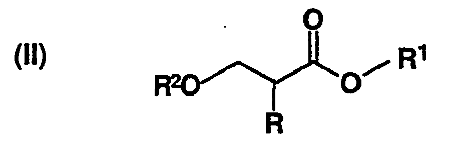

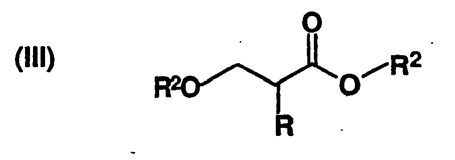

Die Folgen dieser Adduktbildung sind eine verminderte Ausbeute und ein erhöhter Destillationsaufwand um den Zielester in hoher Reinheit zu erlangen.

Die Bildung der Additionsprodukte entsprechend der allgemeinen Gleichung II kann, wie allgemein bekannt ist, dadurch verringert werden, daß die Konzentration an freiem Alkanol möglichst niedrig gehalten wird. EP-A2 906 902 schlägt daher vor, die Hauptmenge des Alkanols während der Umesterung kontinuierlich zuzugeben und die Konzentration des Alkanols dabei nicht über 25 Mol% ansteigen zu lassen.

Des weiteren wird in derselben Schrift vorgeschlagen, die Temperatur bei der destillativen Leichtsieder- und Katalysatorabtrennung im Bereich zwischen 60 und 120 °C zu halten, um die zusätzliche Bildung von Michael-Additionsprodukten zu verringern.

Ein weiteres Problem stellt die Instabilität mancher Titanalkoholate bei höheren Temperaturen dar. Die Folge ist eine Belagsbildung (sog. Fouling) an den Apparatewänden.

Dies ist besonders nachteilig, weil wegen ihrer relativ geringen Aktivität erhöhte Temperaturen in der Umesterung notwendig sind, um wirtschaftliche Umsätze bzw. Reaktionszeiten zu erreichen (EP-A2 160 427, Seite 2, Zeilen 23 bis 30). Dies kann wiederum zu einer vermehrten Polymerisat- und Belagsbildung führen.

Es ist außerdem bekannt, daß (Meth)acrylsäureverbindungen eine große Neigung zur Polymerisation besitzen, ganz besonders, wenn Hitze auf sie einwirkt (s. z.B. Ullmann's Encyclopedia of Industrial Chemistry, 6th ed, 1999 Electronic Release, Kapitel: Acrylic Acid and Derivatives). Vor allem bei der Herstellung und der destillativen Reinigungen sind sie Temperaturen ausgesetzt, die leicht eine unerwünschte Polymerisation auslösen können.

Ein Problem stellt auch der Aktivitätsverlust dar, den Titanalkoholate mit der Zeit erleiden (DE-A 28 05 702, Seite 5, Zeilen 12 bis 21). Um wirtschaftliche Umsätze zu erzielen, muß die Katalysatormenge und/oder die Reaktionszeit erhöht werden. In Hinblick auf die Instabilität der Titanate und die Nebenprodukt- und Polymerisatbildung ist dies bekanntlich nachteilig.

Weiterhin ist allgemein bekannt, daß Alkyltitanate die Polymerisation von (Meth)acrylsäureestern katalysieren und daher zur Bildung von Polymerisat bei der Alkoholyse beziehungsweise Umesterung und der Aufarbeitung des Umesterungsgemisches Anlaß geben (DE-OS 20 08 618, Seite 3) oder die Wirkung von Stabilisatoren beeinflussen können (DE-PS 1 067 806, Spalte 1, Zeilen 25 bis 40).

Verschmutzung der Apparaturen, Verstopfen von Leitungen und Pumpen und die Belegung von Kolonnenböden oder -einbauten und Wärmetauscherflächen ("fouling") sind in der Regel die Folge einer Polymerisatbildung. Das Reinigen der Anlagen ist ein aufwendiger, durch z.B. die Abstellzeiten teurer und z.B. durch die anfallenden Reinigungsabwässer umweltbelastender Vorgang. Die Ausbeute und die verfügbarkeit der Anlagen (Laufzeit) wird dadurch außerdem stark reduziert.

DE-PS 1 067 806 schlägt daher vor, die Umesterung unter Druck und bei 180-250°C durchzuführen, um möglichst kurze Verweilzeiten zu erzielen. Anderseits wird der Einsatz von Stabilisatoren oder Stabilisatorgemischen vorgeschlagen, z.B. Aminophenole, Hydrochinon, Hydrochinonmonomethylether, Phenothiazin etc., gegebenfalls mit einem Zusatz von Sauerstoff bzw. Luft (JP-OS 53449/95). Das Arbeiten unter Druck erfordert jedoch hohen apparativen und sicherheitstechnischen Aufwand und ist daher unwirtschaftlich.

DE-OS 19 65 308 schlägt zur Stabilisierung ein Gemisch aus Phenothiazin und Diethylhydroxylamin vor, wobei aber eine unwirtschaftlich hohe Menge von 1-3% bezüglich Alkanol notwendig ist. Eine vollkommene Vermeidung der Polymerisatbildung ist aber auch dadurch nicht möglich.

Da, wie oben beschrieben, die üblichen Umesterungskatalysatoren die Stabilität des Reaktionsgemisches negativ beeinflussen, wird die Entfernung des Katalysators vor der destillativen Aufarbeitung ausdrücklich empfohlen, z.B. durch Waschen mit Wasser (DE-OS 2 805 702).

Die Katalysatorabtrennung zu Recycling-Zwecken nach der destillativen Entfernung des Methanol/Methylmethacrylat-Azeotrops wird auch in EP-A 902 017 beschrieben.

Die Entfernung des Katalysators, z.B. von Titanalkoholaten, ist jedoch sehr aufwendig, da es eine Filtration nach der hydrolytischen Spaltung des Katalysators erfordert.

DE-A 1 142 868 empfiehlt daher die Verwendung von polymeren Titanaten, die im Reaktionsgemisch nicht löslich sind und auch als Feststoff verwendbar sind. Aufgrund ihrer aufwendigen Herstellung haben diese Katalysatoren keine wirtschaftliche Bedeutung erlangt.

Eine weitere Problematik tritt bei der Herstellung von Dialkylaminoethyl(meth)acrylaten durch Umesterung von Dialkylaminoethanolen auf:

Die Herstellung von Dialkylaminoethanolen erfolgt bekanntlich in der Weise, daß man Ethylenoxid in flüssiger Phase bei 100 bis 150 °C mit den entsprechenden Dialkylaminen umsetzt. Als Nebenprodukte treten dabei im allgemeinen höhere Homologe auf, die destillativ im wesentlichen abtrennbar sind, und Nebenprodukte, wie z.B. Vinyloxyethanol und Ethylenglykol, die destillativ nur sehr schwierig abtrennbar sind und daher in Spuren in den Dialkylaminoethanolen vorhanden sind.

Umesterung dieser Komponenten führt zu Ethylenglykoldi(meth)acrylat und Vinyloxyethyl(meth)acrylat, die bei der Herstellung von Alkylaminoethyl(meth)acrylaten als Nebenkomponenten auftreten können und zwei ungesättigte Gruppen enthalten. Daher wirken diese Nebenkomponenten bei der Polymerisation als Vernetzer. Dies ist äußerst nachteilig, da dadurch die Polymerisation und die Qualität der Polymerisate, z.B. durch Gelbildung, beeinträchtigt wird. Außerdem beeinflussen sie die Lagerstabilität.

Wegen der aufgezeigten Stabilitäts- bzw. Polymerisationsprobleme erfolgt die Aufarbeitung des Umesterungsgemisches bzw. die Isolierung des Zielesters in der Regel in mehreren Destillationsstufen.

EP-A2 906 902 beschreibt beispielsweise ein Verfahren zur Herstellung und Isolierung von Alkylaminoalkyl(meth)acrylaten, das im wesentlichen aus folgenden Stufen besteht:
1. Diskontinuierliche Umesterung in Gegenwart des Katalysators Dibutylzinnoxid und des Stabilisators Phenothiazin, wobei die Hauptmenge des Alkylaminoalkanols nach dem Start der Reaktion in der weise zugegeben wird, daß seine Konzentration im Reaktionsgemisch 25 Mol-% nicht überschreitet.
2. Destillative Abtrennung des bei der Umesterung gebildeten niederen Alkanols als Azeotrop mit dem niederen (Meth)acrylester über eine Kolonne, wobei das Destillat gegebenfalls einer weiteren Destillation unterworfen wird.
3. Behandlung des Destillats, das hauptsächlich aus niederem Alkanol und niederem (Meth)acrylat besteht, mit einem sauren Kationenionenaustauscher. Die basischen Verunreinigungen (Amine), die die Verwendung des Destillats bei der Herstellung des niederen Esters durch Vergiftung des dabei verwendeten Katalysators verhindern, werden dabei abgetrennt.
4. Destillative Auftrennung des Reaktionsgemisches in ein Kopfprodukt, hauptsächlich aus Zielester, niederem Alkanol und Ausgangsprodukten bestehend, und ein Sumpfprodukt, das im wesentlichen Katalysator, Stabilisator, Michael-Additionsprodukte und.Polymere enthält, und unter Umständen bei der Umesterung erneut eingesetzt wird. Verliert der Katalysator seine Aktivität, so wird er entsorgt.
   Alternativ kann die Katalysatorabtrennung zweistufig erfolgen, wobei zuerst der niedere (Meth)acrylester über den Kopf einer Kolonne abgetrennt und wieder der Umesterung zugeführt wird. In einer zweiten Destillationskolonne wird der Zielester und verbliebene Leichtsieder als Kopfprodukt abgetrennt und der katalysatorhaltige Sumpf gegebenfalls wieder bei einer Umesterung eingesetzt.
5. Das den Zielester enthaltende Destillat wird in einer weiteren Destillationsstufe in ein Kopfprodukt (Aminoalkanol, niederer Ester), das wieder bei der Umesterung eingesetzt werden kann, und ein Sumpfprodukt, das den Zielester enthält, aufgetrennt.
6. Aus dem zielesterhaltigen Sumpf wird schließlich in einem weiteren Destillationsschritt (Reindestillation) der Zielester in einer Reinheit von 99,8 % isoliert.
7. Aus dem Sumpfprodukt der Reindestillation, das noch Zielester enthält, wird in einer Destillation, vorzugsweise einer Dünnschichtdestillaton, ein Teil des Zielesters gewonnen und der Leichtsiederdestillation zugeführt. Das Sumpfprodukt wird entsorgt.

Der Nachteil des Verfahrens liegt u. a. darin,
- daß die Umesterung diskontinuierlich erfolgt,
- daß das Dialkylaminoalkanol über einen langen Zeitraum (4 Stunden) verteilt dem Reaktor zugegeben werden muß,
- daß lange Reaktionszeiten (7 - 8 Stunden) benötigt werden, was die Bildung von Nebenprodukten und von Polymerisat begünstigt,
- daß das Azeotrop technisch aufwendig über ein Ionenaustauscherbett gereinigt werden muß, was durch Erfordernis von Spülungen und Regenerierungen umweltbelastend ist,
- daß die Ausbeute gering ist (ca. 33 % bezüglich eingesetztem Dimethylaminoethanol, s. Bsp. III-1) und
- daß die Rückstände nicht aufgearbeitet werden, um Wertprodukte wiederzugewinnen.

EP-A2 960 877 beschreibt ein kontinuierliches Verfahren zur Herstellung von Dialkylaminoalkyl(meth)acrylaten durch Umesterung von Methyl- oder Ethyl(meth)acrylat mit Dialkylaminoalkanolen in Gegenwart von Tetraethyl-, Tetrabutyl- oder Tetra(2-ethylhexyl)titanat. Die Umesterung erfolgt dabei in einem Rührreaktor und die Aufarbeitung des Reaktionsgemisch in folgenden Schritten:
1. Das Reaktionsgemisch wird in einer Destillationseinheit in ein Kopfprodukt, das im wesentlichen den Zielester und die Leichtsieder enthält, und ein Sumpfprodukt, das hauptsächlich aus Schwersiedern, Katalysator und etwas Zielester besteht, aufgetrennt.
2. Das Sumpfprodukt kann gegebenenfalls in einem Dünnschichtverdampfer gereinigt werden, wobei das Destillat wieder der Umesterung zugeführt wird. Der katalysatorhaltige Sumpfablauf wird ausgeschleust.
3. Das den Zielester enthaltende Kopfprodukt wird in einem weiteren Destillationsschritt in eine Leichtsiederfraktion, die in den Reaktor zurückgeführt wird, und ein Sumpfprodukt, vorwiegend Zielester, aufgetrennt
4. In einem weiteren destillativen Reinigungsschritt wird aus dem Sumpfprodukt der Zielester als Kopfprodukt isoliert (Reinheit 99,8 %). Der anfallende Rückstand wird in die Leichtsiederabtrennung zurückgeführt.

Dieses Verfahren hat u. a. folgende Nachteile:
- Die Umesterung erfolgt in einem aufgrund seiner bewegten Teile reparaturanfälligen Rührreaktor
- Die Alkanolkomponente des Katalysator führt zu Verunreinigungen (s. EP-A2 960 877, Seite 2, Zeilen 49 bis 50)
- Keine Verwertung des am Reaktor abgetrennten Destillats, somit Verlust von Wertprodukten
- Keine Verwertung der anfallenden Hochsieder (z.B. Michael-Addukte oder Katalysator).

Der vorliegenden Erfindung lag nun die Aufgabe zugrunde, ein Verfahren zu entwickeln, das frei ist von den genannten Schwächen und folgende Kriterien erfüllt:
1. Die Ausgangsstoffe (Katalysator, Stabilisator, niederer (Meth)acrylsäureester) sollen kostengünstig, problemlos handhabbar und in technischer Menge verfügbar sein.
2. Der Katalysator soll bei erhöhter Temperatur und in Gegenwart geringer Wassermengen stabil sein.
3. Der Aktivitätsverlust des Katalysators soll gering sein und er soll problemlos wiederverwendet werden können.
4. Es soll kein systemfremder Alkohol über den Umesterungskatalysator in die Umesterung eingeschleust werden.
5. Hohe Laufzeiten der Anlage, d.h. möglichst geringe Polymerisationsprobleme und Verwendung möglichst weniger reparaturanfälliger Apparate.
6. Direkte Wiederverwendung bzw. Verwertung des anfallenden Gemisches beziehungsweise Azeotrops aus niederem Alkanol und dem entsprechenden Ester.
7. Weitgehende Rückgewinnung der restlichen Wertprodukte aus den Abfallströmen und aus den Nebenprodukten.
8. Die Umesterung soll vorzugsweise kontinuierlich betrieben werden.
9. Der Zielester soll eine hohe Reinheit (mindestens 99,9 %) aufweisen und im Falle der Herstellung von Dialkylaminoethyl(meth)acrylaten soll die Bildung von Ethylenglykoldi(meth)acrylat und Vinyloxyethyl(meth)acrylat verringert werden, möglichst auf unter 100 ppm.
10. Die Abfallmengen sollen möglichst gering und gut handhabbar sein.
11. Umsatz und Ausbeute sollen hoch sein (> 95 %).
12. Die Verweilzeiten sollen gering sein.
13. Das Gesamtverfahren soll technisch einfach und wirtschaftlich sein.

Es wurde nun ein Verfahren zur Herstellung von (Meth)acrylsäureestern IV durch Umesterung eines (Meth)acrylsäureesters I mit einem Alkohol R²OH, der mindestens ein Kohlenstoffatom mehr aufweist als die Alkoholkomponente (R¹O-) im umzuesternden (Meth)acrylsäureester I, in Gegenwart eines Katalysators oder Katalysatorgemisches, gefunden, in dem der bei der Umesterung freigesetzte niedere Alkanol R¹OH gemeinsam mit einem Teil des niederen (Meth)acrylsäureesters I abgetrennt und ohne einen zusätzlichen Reinigungsschritt in eine Anlage zur Herstellung und/oder Aufarbeitung des (Meth)acrylsäureesters I eingespeist wird und aus dem Reaktionsaustrag der Umesterung entweder zunächst im wesentlichen (Meth)acrylsäureester I abgetrennt und dann destillativ vom verwendeten Katalysator abgetrennt wird (Katalysatorabtrennung), oder zunächst destillativ vom verwendeten Katalysator abgetrennt (Katalysatorabtrennung) und dann im wesentlichen (Meth)acrylsäureester I abgetrennt wird
und dann von dem erhaltenen Gemisch leichter als der (Meth)acrylsäureester IV siedende Komponenten destillativ im wesentlichen abgetrennt werden (Leichtsiederabtrennung) und anschließend der (Meth)acrylsäureester IV reindestilliert wird (Reindestillation), wobei die Katalysatorabtrennung bei Temperaturen von 125 bis 160°C durchgeführt wird.

Des weiteren wurde gefunden, daß die Stabilität der Dialkylaminoethyl(meth)acrylate, die durch basische Umesterung von niederen (Meth)acrylsäureestern mit Dialkylaminoethanolen hergestellt werden, entscheidend vom Gehalt der Nebenprodukte im eingesetzten Dialkylaminoethanol abhängt.

Das erfindungsgemäße Verfahren wird im allgemeinen wie folgt durchgeführt:

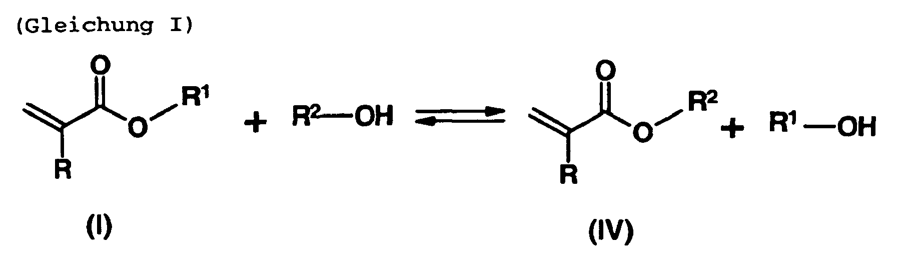

R¹, R² und R³ können jeweils aromatisch, aliphatisch oder cycloaliphatisch, geradkettig oder verzweigt, gesättigt oder ungesättigt sein und Heteroatome oder aromatische Substituenten enthalten.

Bevorzugt umfassen die Reste
R = H, CH₃
R¹ = C₁ - C₄ - Alkyl
R² = C₃ - C₁₂ - Alkyl oder C₂ - C₁₂ - Alkyl, substituiert mit einer
NR³₂ - Gruppe

R³ = C₁ - C₆ - Alkyl, wobei N mit den Substituenten R³ auch einen fünf- bis siebengliedrigen Ring bilden kann und die Substituenten R³ gleich oder verschieden sein können.

R¹ soll dabei mindestens ein Kohlenstoffatom weniger enthalten als R², bevorzugt soll der Siedepunkt des Alkohols R²OH unter den gewählten Reaktionsbedingungen mindestens 20 °C über dem von R¹OH liegen.

Dabei handelt es sich bei R² beispielsweise um n-Propyl-, iso-Propyl-, Allyl-, n-Butyl-, 1-Methyl-propyl-, 2-Methyl-propyl-, tert.-Butyl-, n-Pentyl-, 1-Methyl-butyl-, 2-Methyl-butyl-, 3-Methyl-butyl-, 2,2-Dimethylpropyl-, n-Hexyl-, 1-Methyl-pentyl-, 2-Methyl-pentyl-, 3-Methyl-pentyl-, 4-Methyl-pentyl-, 1,1-Dimethyl-butyl-, 2,2-Dimethyl-butyl-, 3,3-Dimethyl-butyl-, 1,2-Dimethyl-butyl-, n-Heptyl-, 1-Methyl-hexyl-, 2-Methyl-hexyl-, 3-Methyl-hexyl-, 4-Methyl-hexyl-, 1,2-Dimethyl-pentyl-, 1,3-Dimethyl-pentyl-, 1,1-Dimethyl-pentyl-, 1,1,2,2-Tetramethyl-propyl-, Benzyl-, n-Octyl-, 2-Ethylhexyl-, n-Nonyl-, 1-Methyl-octyl-, 2-Methyl-octyl-, n-Decyl-, n-Undecyl-, 1-Methyl-decyl-, 2-Methyl-decyl-, n-Dodecyl-, 2,4-Diethyl-octyl-, Cyclopentyl-, Cyclohexyl-, 4-*tert*.-Butyl-cyclohexyl-, Cycloheptyl-, Cyclododecyl-, 2-(Dimethylamino)-ethyl-, 3-(Dimethylamino)-propyl-, 4-(Dimethylamino)-butyl-, 5-(Dimethylamino)-pentyl-, 6-(Dimethylamino)-hexyl-, 8-(Dimethylamino)-octyl-, 10-(Dimethylamino)-decyl-, 12-(Dimethylamino)-dodecyl-, 2-(Diethylamino)-ethyl-, 3-(Diethylamino)-propyl-, 4-(Diethylamino)-butyl-, 5-(Diethylamino)-pentyl-, 6-(Diethylamino)-hexyl-, 8-(Diethylamino)-octyl-, 10-(Diethylamino)-decyl-, 12-(Diethylamino)-dodecyl-, 2-(Di-(iso-propyl)-amino)-ethyl-, 3-(Di-(iso-propyl)-amino)-propyl-, 4-(Di-(iso-propyl)-amino)-butyl-, 5-(Di-(iso-propyl)-amino)-pentyl-, 6-(Di-(iso-propyl)-amino)-hexyl-, 8-(Di-(iso-propyl)-amino)-octyl-, 10-(Di-(iso-propyl)-amino)-decyl-, 12-(Di-(iso-propyl)-amino)-dodecyl-, 2-(Dibutylamino)-ethyl-, 3-(Dibutylamino)-propyl-, 4-(Dibutylamino)-butyl-, 5-(Dibutylamino)-pentyl-, 6-(Dibutylamino)-hexyl-, 8-(Dibutylamino)-octyl-, 10-(Dibutylamino)-decyl-, 12-(Dibutylamino)-dodecyl-, 2-(Dihexylamino)-ethyl-, 3-(Dihexylamino)-propyl-, 4-(Dihexylamino)-butyl-, 5-(Dihexylamino)-pentyl-, 6-(Dihexylamino)-hexyl-, 8-(Dihexylamino)-octyl-, 10-(Dihexylamino)-decyl-, 12-(Dihexylamino)-dodecyl-, 2-(Methyl-ethyl-amino)-ethyl-, 2-(Methyl-propylamino)-ethyl-, 2-(Methyl-iso-propyl-amino)-ethyl-, 2-(Methyl-butyl-amino)-ethyl-, 2-(Methyl-hexyl-amino)-ethyl-, 2-(Methyl-octyl-amino)-ethyl-, 2-(Ethyl-propyl-amino)-ethyl-, 2-(Ethyl-iso-propyl-amino)-ethyl-, 2-(Ethyl-butyl-amino)-ethyl-, 2-(Ethyl-hexyl-amino)-ethyl-, 2-(Ethyl-octyl-amino)-ethyl-, 3-(Methyl-ethylamino)-propyl-, 3-(Methyl-propyl-amino)-propyl-, 3-(Methyl-iso-propyl-amino)-propyl-, 3-(Methyl-butyl-amino)-propyl-, 3-(Methyl-hexyl-amino)-propyl-, 3-(Methyl-octyl-amino)-propyl-, 3-(Ethylpropyl-amino)-propyl-, 3-(Ethyl-iso-propyl-amino)-propyl-, 3-(Ethyl-butyl-amino)-propyl-, 3-(Ethyl-hexyl-amino)-propyl-, 3-(Ethyl-octyl-amino)-propyl-, 4-(Methyl-ethyl-amino)-butyl-, 4-(Methyl-propyl-amino)-butyl-, 4-(Methyl-iso-propylamino)-butyl-, 4-(Methyl-butyl-amino)-butyl-, 4-(Methyl-hexylamino)-butyl-, 4-(Methyl-octyl-amino)-butyl-, 4-(Ethyl-propylamino)-butyl-, 4-(Ethyl-iso-propyl-amino)-butyl-, 4-(Ethyl-butylamino)-butyl-, 4-(Ethyl-hexyl-amino)-butyl-, 4-(Ethyl-octylamino)-butyl-, 2-(N-Piperidinyl)-ethyl-, 3-(N-Piperidinyl)-propyl-, 4-(N-Piperidinyl)-butyl-, 5-(N-Piperidinyl)-pentyl-, 6-(N-Piperidinyl)-hexyl-, 8-(N-Piperidinyl)-octyl-, 10-(N-Piperidinyl)-decyl-, 12-(N-Piperidinyl)-dodecyl-, 2-(N-Pyrrolidinyl)-ethyl-, 3-(N-Pyrrolidinyl)-propyl-, 4-(N-Pyrrolidinyl)-butyl-, 5-(N-Pyrrolidinyl)-pentyl-, 6-(N-Pyrrolidinyl)-hexyl-, 8-(N-Pyrrolidinyl)-octyl-, 10-(N-Pyrrolidinyl)-decyl-, 12-(N-Pyrrolidinyl)-dodecyl-, 2-(N-Morpholino)-ethyl-, 3-(N-Morpholino)-propyl-, 4-(N-Morpholino)-butyl-, 5-(N-Morpholino)-pentyl-, 6-(N-Morpholino)-hexyl-, 8-(N-Morpholino)-octyl-, 10-(N-Morpholino)-decyl-, 12-(N-Morpholino)-dodecyl-, 2-(N'-Methyl-N-Piperazinyl)-ethyl-, 3-(N'-Methyl-N-Piperazinyl)-propyl-, 4-(N'-Methyl-N-Piperazinyl)-butyl-, 5-(N'-Methyl-N-Piperazinyl)-pentyl-, 6-(N'-Methyl-N-Piperazinyl)-hexyl-, 8-(N'-Methyl-N-Piperazinyl)-octyl-, 10-(N'-Methyl-N-Piperazinyl)-decyl-, 12-(N'-Methyl-N-Piperazinyl)-dodecyl-, 2-(N'-Ethyl-N-Piperazinyl)-ethyl-, 3-(N'-Ethyl-N-Piperazinyl)-propyl-, 4-(N'-Ethyl-N-Piperazinyl)-butyl-, 5-(N'-Ethyl-N-Piperazinyl)-pentyl-, 6-(N'-Ethyl-N-Piperazinyl)-hexyl-, 8-(N'-Ethyl-N-Piperazinyl)-octyl-, 10-(N'-Ethyl-N-Piperazinyl)-decyl-, 12-(N'-Ethyl-N-Piperazinyl)-dodecyl-, 2-(N'-*iso*-Propyl-N-Piperazinyl)-ethyl-, 3-(N'-*iso*-Propyl-N-Piperazinyl)-propyl-, 4-(N'-*iso*-Propyl-N-Piperazinyl)-butyl-, 5-(N'-*iso*-Propyl-N-Piperazinyl)-pentyl-, 6-(N'-*iso*-Propyl-N-Piperazinyl)-hexyl-, 8-(N'-*iso*-Propyl-N-Piperazinyl)-octyl-, 10-(N'-*iso*-Propyl-N-Piperazinyl)-decyl-, 12-(N'-*iso*-Propyl-N-Piperazinyl)-dodecyl-, 3-Oxa-butyl-, 3-Oxapentyl-, 2,2-Dimethyl-4-oxa-pentyl-, 3,6-Dioxa-heptyl-, 3,6-Dioxa-octyl-, 3,6,9-Trioxa-decyl-, 3,6,9-Trioxa-undecyl-, 4-Oxa-pentyl-, 4-Oxa-hexyl-, 4-Oxa-heptyl-, 4,8-Dioxa-nonyl-, 4,8-Dioxadecyl-, 4,8-Dioxa-undecyl-, 5-Oxa-hexyl- oder 5,10-Dioxa-undecyl-.

Weiterhin kann es sich bei R²OH um ethoxylierte und/oder propoxylierte Alkohole sowie gemischt-ethoxylierte/propoxylierte Alkohole wie
R⁵-(O-CH₂-CH₂)ₓ-OH oder
R⁵-(O-CH(CH₃)-CH₂)ₓ-OH beziehungsweise R⁵-(O-CH₂-CH(CH₃))ₓ-OH,
worin
R⁵ für C₁ bis C₂₀-Alkyl und
x für eine ganze Zahl zwischen 1 und 20
steht,
oder um ethoxylierte und/oder propoxylierte Aminoalkohole
R³₂N(-CH₂CH₂-O)_{y}-H oder
R³₂N(-CH(CH₃)-CH₂-O)_{y}-H beziehungsweise R³₂N(-CH₂-CH(CH₃)-O-)_{y}-H,
worin y für eine ganze Zahl zwischen 1 und 4 steht,
handeln.

Vorzugsweise werden Dialkylaminoethanole eingesetzt, besonders bevorzugt sind Dimethylaminoethanol, Diethylaminoethanol und Din-butylaminoethanol.

In einer besonders bevorzugten Ausführungsform werden Dialkylaminoethanole mit einem Gehalt an Ethylenglykol von nicht mehr als 100 ppm, bevorzugt nicht mehr als 50 ppm, besonders bevorzugt nicht mehr als 20 ppm und insbesondere nicht mehr als 10 ppm eingesetzt.

In einer weiteren besonders bevorzugten Ausführungsform werden Dialkylaminoethanole mit einem Gehalt an Vinyloxyethanol von nicht mehr als 100 ppm, bevorzugt nicht mehr als 50 ppm, besonders bevorzugt nicht mehr als 20 ppm und insbesondere nicht mehr als 10 ppm eingesetzt.

Für das erfindungsgemäße Verfahren sind sämtliche im Stand der Technik beschriebenen Umesterungskatalysatoren geeignet, vorzugsweise Titan-, Magnesium- oder Aluminiumalkoholate, besonders bevorzugt Titanalkoholate und insbesondere Titanalkoholate der Alkohole, die in der Umesterung vorliegen, also R¹OH und R²OH.

### 1. Katalysatorherstellung

Im Fall der Verwendung von Titanalkoholaten wird ein niederes Titanalkoholat Ti(OR⁴)₄, vorzugsweise das Isopropylat, Isobutylat bzw. n-Butylat, mit dem höheren Alkohol R²OH (siehe Gleichung III) bei erhöhter Temperatur (50 - 130 °C) zur Reaktion gebracht. Dabei wird der höhere Alkohol in molarem Überschuß (in der Regel 1:5 bis 1:20) eingesetzt.

Gleichung III

**Ti(OR**^{**4**}**)**_{**4**} **+ R**^{**2**}**OH ⇄ Ti(OR**^{**2**}**)**_{**4**} **+ R**^{**4**}**OH**

R² s. Gleichung (I)
R⁴ steht für C₁ - C₈ - Alkyl, vorzugsweise für Isopropyl, Isobutyl- oder n-Butyl
R²OH und R⁴OH sollen dabei über ihre Siedepunkte Kp vorzugsweise folgende Bedingung erfüllen:

Kp. (R²OH) ≥ Kp. (R⁴OH) + 20 °C

Unter diesen Bedingungen ist es technisch einfach, die Verluste an R²OH gering zu halten und R⁴OH möglichst vollständig abzutrennen.

Der bei der Reaktion entstehende Alkohol R⁴OH wird, gegebenenfalls bei vermindertem Druck, destillativ oder rektifikativ abgetrennt. Dies kann gegebenenfalls durch Strippen mit einem geeigneten reaktionsträgen Gas unterstützt werden. Der anfallende Rückstand stellt die Katalysatorlösung für die Umesterung dar (Ti - Gehalt: 2 - 10 Gew%) und enthält weniger als 400 ppm R⁴OH. Es wird somit praktisch kein Fremdalkohol (R⁴OH) in das Umesterungsgemisch (< 100 ppm im Gemisch) eingeschleust.

Selbstverständlich können in der Katalysatorlösung jedoch auch gemischte Titanalkoholate enthalten sein, in Abhängigkeit der Umsetzung nach Gleichung III.

Das Destillat, vorwiegend der Alkohol R⁴OH, kann vorteilhaft zur Verdünnung des anfallenden Rückstandes (s. unten, Stufe 9) genutzt werden.

### 2. Umesterung

Der eingesetzte niedere (Meth)acrylsäureester I hat in der Regel folgende Zusammensetzung:

| | |
|---|---|
| 99,0 - 99,95 Gew% | (Meth)acrylsäureester |
| 0,001 - 0,1 Gew% | Essigsäureester |
| 0,02 - 0,1 Gew% | Propionsäureester |
| 0,001 - 0,05 Gew% | Wasser |

Weiterhin können das niedere Alkanol R¹OH, dessen Dialkylether, (Meth)acrylsäure sowie andere, z.B. isomere (Meth)acrylsäureester enthalten sein.

Der höhere Alkohol R²OH hat üblicherweise eine Reinheit von min. 99,0 Gew% und einen Wassergehalt von 0,01 - 0,2 Gew%.

Im Fall von Dialkylaminoethanolen können auch Spuren an höheren Homologen enthalten sein.

Der Gehalt an Ethylenglykol soll nicht mehr als 100 ppm betragen, bevorzugt nicht mehr als 50 ppm, besonders bevorzugt nicht mehr als 20 ppm und insbesondere nicht mehr als 10 ppm. Der Gehalt an Vinyloxyethanol soll nicht mehr als 100 ppm betragen, bevorzugt nicht mehr als 50 ppm, besonders bevorzugt nicht mehr als 20 ppm und insbesondere nicht mehr als 10 ppm.

Es können auch Gemische von höheren Alkoholen zur Umesterung verwendet werden.

Der niedere (Meth)acrylsäureester I, vorzugsweise der Methyl- oder Ethylester, wird mit dem.höheren Alkohol R²OH in einem molaren Verhältnis von Ester : Alkohol 1:1 bis 4:1 in Gegenwart der unter "1" zubereiteten Katalysatorlösung umgesetzt, wobei der Ti-Gehalt im Reaktionsgemisch in der Regel 0,01 - 1 Gew% beträgt.

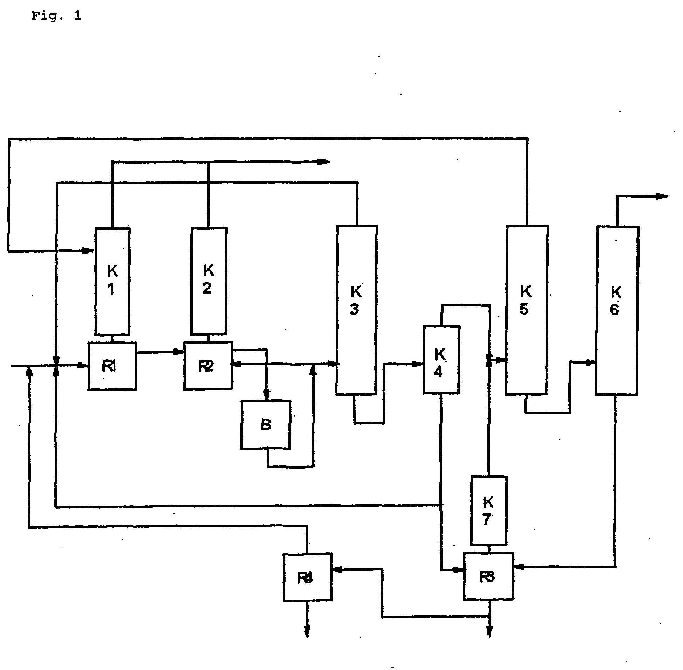

Die Umesterung erfolgt in einem oder mehreren, bevorzugt in ein bis zwei in Serie geschaltenen Reaktoren (R1, R2) mit aufgesetzten Rektifikationskolonnen (K1, K2, siehe Figur 1) und Kondensatoren.

Die gleichmäßige Durchmischung der Reaktionslösung erfolgt auf bekannte Weise, z.B. durch Rühren, Umpumpen oder Naturumlauf, vorzugsweise durch Natur- oder Zwangsumlauf (in Figur 1 nicht dargestellt).

Die Reaktionstemperatur beträgt in der Regel 80 - 140°C, bevorzugt 100 bis 130 °C, der Druck 200 mbar bis Atmosphärendruck, bevorzugt 300 - 800 mbar und besonders bevorzugt 400 bis 600 mbar.

Die Gesamtverweilzeit beträgt im allgemeinen 1 - 4, bevorzugt 2 bis 4 Stunden.

Die Wärmezufuhr kann über eine Wandbeheizung oder/und außen- oder innenliegende Wärmetauscher, z. B. Röhren- oder Plattenwärmetauscher, erfolgen.

Die Reaktionszone kann bevorzugt mit einem unter den Reaktionsbedingungen inerten Gas oder Gasgemisch, z.B. Stickstoff, Luft, Luft-Stickstoff- oder Stickstoff - Sauerstoff - Gemische, Argon, Helium, Kohlenstoffdi- oder -monooxid, kontinuierlich gespült werden. Besonders bevorzugt wird das Spülgas entlang der vorhandenen Wärmetauscherflächen geleitet, insbesondere in einem vorhandenen Umpump- oder Naturumlaufkreislauf, wie in der deutschen Patentanmeldung mit dem Titel "Verfahren zur Herstellung von (Meth)acrylsäureestern" und dem gleichen Anmeldetag wie die vorliegene Schrift und dem Aktenzeichen 101 27 938.8 beschrieben ist.

Das Umesterungsgemisch (Reaktoraustrag) wird in einen isolierten Behälter (B) befördert, der als Pufferbehälter für die anschließende Destillationseinheit (K3) dient.

Eine besonders bevorzugte Ausführungsform der Stufe "2" besteht darin, daß der Austrag des Zwischenbehälters (B) kontinuierlich wieder in einen der Umesterungsreaktoren (R1 beziehungsweise R2, vorzugsweise in R2) zurückgeführt und lediglich ein Teilstrom (10 - 75 %) der anschließenden Destillation K3 zugeführt wird.

In einer weiteren besonders bevorzugten Ausführungsform sei der Behälter B z.B. auf 50 bis 140 °C beheizbar und stehe mit dem Gasraum von R2 und/oder K2 in Verbindung. Die Verweilzeit im Behälter (B) beträgt in der Regel 0,5 - 1,5 Stunden. Auf diese Weise wird in (B) restlicher höherer Alkohol (R²OH) umgesetzt und das dabei entstehende niedere Alkanol (R¹OH) über R2 und K2 abgetrennt und aus dem Gleichgewicht entfernt. Der Vorteil ist ein höherer Umsatz bei geringem Aufwand (technisch und energetisch).

### 3. Abtrennung des niederen Alkanols

Die Rektifikationskolonnen sind von bekannter Bauart und haben trennwirksame Einbauten (z. B. Glocken-, Sieb- oder Dual-Flow-Böden) oder enthalten Schüttungen oder gerichtete Packungen.

Die dem/den Reaktor(en) aufgesetzte(n) Kolonne(n) hat/haben in der Regel 10 - 30 theoretische Böden. Das Rücklaufverhältnis liegt in der Regel bei 5 - 15 : 1, vorzugsweise bei 7 - 12 : 1.

Die Kondensatoren sind ebenfalls von bekannter Bauart, z. B. Röhren- oder Plattenwärmetauscher.

Das bei der Umesterung in den Reaktoren R1 und R2 freigesetzte niedere Alkanol R¹OH wird gemeinsam mit einem Teil des niederen (Meth)acrylsäureesters I über den Kopf der den Reaktoren aufgesetzten Rektifikationskolonnen (K1, K2) abgetrennt.

Die Destillationsbedingungen, z.B. die Trennstufen und das Rücklaufverhältnis, werden dabei so gewählt, daß am Kopf der Kolonne ein nichtazeotropes Gemisch abgenommen wird, bei dem gegenüber der azeotropen Zusammensetzung aus niederem Alkanol und niederem (Meth)acrylsäureester der Gehalt an niederem (Meth)acrylsäureester erhöht ist.

Die Destillationsbedingungen werden so eingestellt, daß der Alkanolgehalt im Kondensat beispielsweise im Falle von Methanol 20 - 40 Gew. %, im Falle von Ethanol 30 - 65 Gew. %, vorzugsweise 40 bis 60 Gew.% beträgt. In der Regel sind nicht mehr als 1, bevorzugt nicht mehr als 0,5 und besonders bevorzugt nicht mehr als 0,3 Gew.% des höheren Alkohols R²OH enthalten.

Dies hat den Vorteil, daß weniger Trennstufen und weniger Rücklauf, damit geringerer Energieverbrauch, erforderlich ist, und daurch eine geringere thermische Belastung und geringeres Fouling, insbesondere der Verdampfer resultiert, so daß längere Anlagenlaufzeiten durch weniger Abstellungen erzielt werden können.

Ein Teil des Kondensats kann als Rücklauf wieder auf den Kolonnenkopf aufgebracht werden, beispielsweise 40 - 99%, bevorzugt 70 - 95% (in Figur 1 nicht dargestellt).

Der restliche Teil des Kondensats wird direkt, d. h. ohne einen zusätzlichen Reinigungsschritt, in die Synthese des niederen Alkyl(meth)acrylats I zurückgeführt, wo es mit (Meth)acrylsäure wieder zum Ausgangsester umgesetzt werden kann, wie es beispielsweise in der deutschen Patentanmeldung mit dem Titel "Verfahren zur Herstellung von (Meth)acrylsäureestern" mit dem gleichen Anmeldetag wie die vorliegende Schrift und dem Aktenzeichen 101 27 941.8 beschrieben ist. Vorteilhaft kann es dort dem Aufarbeitungsprozeß zugeführt werden, besonders bevorzugt einem Extraktionsprozeß.

Die Stabilisierung der Kolonne kann mit den gängigen Stabilisatoren oder Gemischen davon erfolgen, wie z.B. N-Oxyle, wie z.B. 4-Hydroxy-2,2,6,6-tetramethylpiperidin-N-Oxyl oder 4-Oxo-2,2,6,6-tetramethylpiperidin-N-Oxyl, Phenole und Naphthole, wie z.B. p-Aminophenol, p-Nitrosophenol, 2-*tert*.-Butylphenol, 4-*tert*.-Butylphenol, 2,4-di-*tert*.-Butylphenol, 2-Methyl-4-*tert*.-Butylphenol, 4-Methyl-2,6-*tert*.-Butylphenol (2,6-*tert*.-Butyl-p-Kresol) oder 4-*tert*.-Butyl-2,6-dimethylphenol, Chinone, wie z.B. Hydrochinon oder Hydrochinonmonomethylether, aromatische Amine, wie z.B. N,N-Diphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B. N,N-Diethylhydroxylamin, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin. Weiterhin können dies auch Abbauprodukte oder Derivate von Stabilisatoren sein, beispielsweise das Michael-Addukt von (Meth)acrylsäure beziehungsweise (Meth)acrylsäureester und Hydrochinon.

Die Stabilisierung kann in An- oder Abwesenheit von molekularem Sauerstoff erfolgen.

Bevorzugt erfolgt die Stabilisierung mit Phenothiazin, Hydrochinon, Hydrochinonmonomethylether, 4-Hydroxy-2,2,6,6-tetramethylpiperidin-N-Oxyl, 4-Oxo-2,2,6,6-tetramethylpiperidin-N-Oxyl, 2,6-*tert*.-Butyl-p-Kresol oder Gemischen davon in Mengen von jeweils zwischen 10 und 5000 ppm. Die Zugabe kann jeweils über die Ausgangsstoffe oder über die Rückführ- oder Rücklaufströme erfolgen.

Besonders bevorzugt erfolgt die Stabilisierung mit dem mit 100-1000 ppm Phenothiazin und 10 - 500 ppm 4-Hydroxy-2,2,6,6-tetramethylpiperidin-N-Oxyl versetzten Rücklauf. Vorzugsweise erfolgt die Stabilisierung durch Zugabe einer Lösung dieses Stabilisatorgemisches im niederen (Meth)acrylsäureester.

In besonders bevorzugt Weise wird das gelöste Stabilisatorgemisch auf Kondensatorflächen aufgesprüht.

### 4. Abtrennung des niederen (Meth)acrylsäureesters

Das Reaktionsgemisch wird einer Destillation oder Rektifikation (K3) unterworfen, in der im wesentlichen das restliche niedere Alkanol R¹OH und die Hauptmenge, z.B. ca. 80 - 90%, des noch vorhandenen niederen (Meth)acrylsäureesters I abgetrennt werden.

Die Rektifikationskolonne ist von bekannter Bauart und hat trennwirksame Einbauten (z. B. Glocken-, Sieb- oder Dual-Flow-Böden) oder enthält Schüttungen oder gerichtete Packungen.

Die Kolonne hat in der Regel 10 - 30 theoretische Böden. Das Rücklaufverhältnis liegt in der Regel bei 1 : 3 bis 1 : 15, vorzugsweise bei 1 : 5 bis 1 : 10.

Die Kondensatoren und Verdampfer sind ebenfalls von bekannter Bauart, z. B. Röhren- oder Plattenwärmetauscher.

Das Durchmischen erfolgt auf bekannte Weise, z.B. durch Rühren, Umpumpen oder Naturumlauf, vorzugsweise durch Natur- oder Zwangsumlauf (in Figur 1 nicht dargestellt).

Die Sumpftemperatur beträgt im allgemeinen 100 - 170 °C, bevorzugt 120 - 160 °C und besonders bevorzugt 130 - 150°C. Es hat sich gezeigt, daß sich diese Temperaturen vorteilhaft auf die Bildungsrate der Michael-Addukte auswirken, wie sie in der EP-A 906 902, Seite 9, Zeilen 21 bis 28 beschrieben sind.

Der Druck beträgt im allgemeinen zwischen 20 mbar und normalem Druck, bevorzugt 50 - 800 mbar und besonders bevorzugt 100 - 500 mbar.

Für die Stabilisierung gilt das unter "3" Gesagte. Das mit 100 - 1000 ppm Phenothiazin und 10 - 500 ppm 4-Hydroxy-2,2,6,6-tetramethylpiperidin-N-Oxyl stabilisierte (s. o.) Kondensat, kann teilweise, z.B. 5 - 20%, als Rücklauf wieder der Kolonne zugeführt werden. Der Rest kann bevorzugt dem Umesterungsreaktor (R1) zugeführt werden, z.B. über die aufgesetzte Kolonne K1 oder direkt in den Reaktor R1.

### 5. Katalysatorabtrennung

Das vom niederen (Meth)acrylsäurester weitgehend befreite Umesterungsgemisch wird einer Destillation oder Rektifikation, bevorzugt einer Dünnfilm- oder Flash-Verdampfung, besonders bevorzugt eine Flash-Verdampfung (K4), gegebenenfalls unter Anwendung eines Strippgases, bei 125 - 160°C und bevorzugt bei 130 - 150°C, und einem Druck von im allgemeinen 10 - 500 mbar, bevorzugt 20 - 300 mbar und besonders bevorzugt 20 - 200 mbar unterzogen.

In einer besonders bevorzugt Ausführungsform ist der Flash-Verdampfer zusätzlich mit einem Umlaufverdampfer ausgerüstet.

Dabei destillieren im wesentlichen der Zielester IV und Leichtsieder (z.B. Ausgangsstoffe) ab und ein Sumpf fällt an, der hauptsächlich aus Katalysator, Stabilisatoren und Michael-Additionsprodukten besteht.

Dieses Sumpfgemisch kann teilweise, bevorzugt zu 60 - 95%, in die Umesterung zurückgeführt und der Rest einer nicht beschränkten Rückstandsbehandlung (R3) zugeführt werden, in der noch vorhandener Zielester zurückgewonnen wird und gleichzeitig die Michael-Additionsprodukte, wie z.B. II und III, in die entsprechenden Alkohole und (Meth)acrylsäureester zurückgespalten und abgetrennt werden.

Es kann auch sinnvoll sein, die Schritte "4" und "5" zu vertauschen, d.h. den Austrag aus der Umesterung zunächst in einem wie unter "5" beschriebenen Apparat vom Sumpf, der hauptsächlich aus Katalysator, Stabilisator und Michael-Additionsprodukten besteht, zu trennen und anschließend im wesentlichen das niedere Alkanol und den niederen (Meth)acrylsäureester in einer wie unter "4" beschriebenen Destillation oder Rektifikation abzutrennen.

In diesem Fall wird der Sumpf aus K4 wie zuvor in die Rückstandsbehandlung R3 geleitet und der Sumpf aus K3, gegebenenfalls zusammen mit dem Destillat aus K7, in K5 geleitet (Leichtsiederabtrennung). Dies hat den Vorteil, daß das Reaktionsgemisch kürzer dem Katalysator ausgesetzt ist, so daß katalysatorinduzierte Neben- oder Folgereaktionen vermindert werden.

Je nach Art des niederen Esters kann es vorteilhaft sein, den Schritt "5" vorzuziehen und die Schritte "4" und "6" zu vereinigen, für den Methyl- und Ethyl(meth)acrylsäureester als niederen Ester werden die Schritte jedoch in der Reihenfolge "4", "5" und "6" durchlaufen bevorzugt.

### 6. Leichtsiederabtrennung

Das unter "5" anfallende, vom Katalysator abgetrennte Destillat, hauptsächlich aus 85 - 95 % Zielester IV, 5 - 15 % höherem Alkohol R²OH, 1 - 2 % niederem (Meth)acrylsäureester I und 1 - 2 % Michael-Additionsprodukten wie z.B. II oder III bestehend, wird gemeinsam mit dem Destillat der Rückstandsbehandlung (K7) in einem weiteren Destillationsschritt (K 5) in ein Destillat, bestehend aus niederem und höherem (Meth)acrylsäureester und höherem Alkohol, und ein Sumpfprodukt, hauptsächlich aus dem Zielester bestehend, aufgetrennt.

Die Rektifikationskolonne ist von bekannter Bauart und hat trennwirksame Einbauten (z. B. Glocken-, Sieb- oder Dual-Flow-Böden) oder enthält Schüttungen oder gerichtete Packungen.

Die Kolonne hat in der Regel 10 - 30 theoretische Böden. Das Rücklaufverhältnis liegt in der Regel bei 10 : 1 bis 1 : 10, vorzugsweise bei 1 : 2 bis 5 : 1.

Die Kondensatoren und Verdampfer sind ebenfalls von bekannter Bauart, z. B. Röhren- oder Plattenwärmetauscher.

Das stabilisierte Destillat kann einerseits (zu beispielsweise 20 - 50%) in den Reaktor R1 und/oder R2 und/oder über die dem Reaktor aufgesetzte Kolonne (K1 und/oder K2) wieder der Umesterung zugeführt werden, wobei sich bevorzugt der Zulauf in der unteren Hälfte der Kolonne befindet, anderseits als Rücklauf wieder der Destillationskolonne zugeführt werden.

Für die Stabilisierung gilt dabei das unter "3" Gesagte, bevorzugt wird mit 100 - 1000 ppm Phenothiazin und 10 - 500 ppm 4-Hydroxy-2,2,6,6-tetramethylpiperidin-N-Oxyl stabilisiert.

### 7. Reinesterdestillation

Aus dem unter "6" anfallendem Sumpf wird der Zielester IV in einem weiteren Destillationsschritt (K 6) über den Kopf der Kolonne abdestilliert.

Die Rektifikationskolonne ist von bekannter Bauart und hat trennwirksame Einbauten (z. B. Glocken-, Sieb- oder Dual-Flow-Böden) oder enthält Schüttungen oder gerichtete Packungen.

Die Kolonne hat in der Regel 10 - 30 theoretische Böden. Das Rücklaufverhältnis liegt in der Regel bei 10 : 1 bis 1 : 10, vorzugsweise bei 1 : 2 bis 5 : 1.

Die Kondensatoren und Verdampfer sind ebenfalls von bekannter Bauart, z. B. Röhren- oder Plattenwärmetauscher.

Die Kondensation kann auch durch einen mit stabilisiertem Reinprodukt betriebenem Quench üblicher Bauart (bei einer Quenchtemperatur von 20 bis 40 °C) betrieben werden

Der Zielester, d.h. der höhere (Meth)acrylsäureester IV, wird mit einem Lagerstabilisator, z.B. Hydrochinon, Hydrochinonmonomethylether, 4-Hydroxy-2,2,6,6-tetramethylpiperidin-N-Oxyl, 4-Oxo-2,2,6,6-tetramethylpiperidin-N-Oxyl, 2,6-*tert*.-Butyl-p-Kresol oder Gemische davon, bevorzugt 2,6-*tert*.-Butyl-p-Kresol und/oder Hydrochinonmonomethylether, besonders bevorzugt Hydrochinonmonomethylether in Mengen von jeweils 5 - 500 ppm, bevorzugt 10 - 300 ppm und besonders bevorzugt 10 - 200 ppm stabilisiert.

10 - 50% des Destillats können als Rücklauf wieder auf den Kopf der Kolonne aufgebracht werden (in Figur 1 nicht dargestellt).

Das Sumpfprodukt, hauptsächlich aus restlichem Zielester, Michael-Additionsprodukten, Stabilisator und Polymeren bestehend, kann zumindest teilweise gemeinsam mit dem Sumpf aus "5" der Rückstandsbehandlung zugeführt werden.

Es ist auch denkbar, die Schritte "6" und "7" zu vereinigen, wobei das den Zielester enthaltende Destillat aus "5", gegebenenfalls mit dem Destillat aus der Rückstandsbehandlung vereinigt, in einer weiteren Destillationseinheit in eine Leichtsiederfraktion, die hauptsächlich aus den niederem und höherem (Meth)acrylsäureester und höherem Alkohol besteht und in die Umesterung zurückgeführt werden kann, in eine Schwersiederfraktion (Sumpf), die hauptsächlich aus Zielester, Michael-Addukten und Inhibitoren besteht und vorteilhaft in die Rückstandsbehandlung geführt wird, und eine Mittelsiederfraktion, die im wesentlichen den Zielester enthält, aufgetrennt werden kann. Der Zielester wird, vorzugsweise gasförmig, über einen Seitenabzug im unteren Kolonnenbereich, bevorzugt in der unteren Hälfte, besonders bevorzugt im unteren Drittel, ausgeschleust, kondensiert und wie oben beschrieben stabilisiert.

Die Sumpftemperatur beträgt in der Regel 110 - 130 °C, der entsprechende Druck 100 - 200 mbar.

### 8. Rückstandsbehandlung

Die vereinigten Sümpfe der Stufen 5 und 7 werden in einem Reaktor (R3), gegebenenfalls mit aufgesetzter Kolonne (K7), auf 100 - 220 °C, bevorzugt 120 - 200 °C und besonders bevorzugt 130 - 180 °C, gegebenenfalls in Gegenwart eines sauren oder basischen Katalysators, wie z.B. Schwefelsäure, Phosphorsäure, Sulfonsäuren, wie para-Toluolsulfonsäure oder Dodecylsulfonsäure, sauren Metalloxiden oder Ionentauschern oder Metallalkoholate, Alkalimetallcarbonate oder Alkalimetallhydroxide, vorzugsweise ohne zusätzlichen Katalysator erhitzt. Bei dieser Temperatur erfolgt bereits eine Rückspaltung der Michael-Additionsprodukte in die zugrundeliegenden Bestandteile Alkohol und Ester (Rückreaktion zu Gleichung II). Die Spaltrate beträgt unter den genannten Reaktionsbedingungen im allgemeinen etwa 50 Gew% oder mehr.

In einer bevorzugten Ausführungsform können die Spaltprodukte über K7, gegebenenfalls unter Anwendung eines Strippgases, abgetrennt und der Leichtsiederdestillation (K5) oder der Umesterung R1 beziehungsweise R2 und/oder K1 beziehungsweise K2 wieder zugeführt werden.

Es sind daher keine speziellen, technisch aufwendigen Maßnahmen notwendig, um die Bildung der Michael-Additionsprodukte während der Synthese und/oder während der Aufarbeitung zu minimieren.

### 9. Rückstandsaufarbeitung

Der Austrag der Rückstandsbehandlung, der hauptsächlich Polymere, Stabilisatoren und den Katalysator (z.B. das Titanat des höheren Alkohols) enthält, wird bei 50 - 180 °C, bevorzugt 70 - 170 °C und besonders bevorzugt bei 80 - 150 °C mit einer hochsiedenden, Hydroxylgruppen enthaltenden Substanz in R4 umgesetzt. Dabei handelt es sich bevorzugt um preiswerte Mono- oder Polyalkohole, gegebenfalls einem Gemisch davon oder einem Rückstand, der diese enthält.

Hochsiedend sind solche Hydroxylgruppen enthaltenden Substanzen, deren Siedepunkt bei Normaldruck mindestens 200 °C beträgt.

Die dabei durch Umesterung freigesetzten Alkohole (R²OH und R¹OH) werden bevorzugt kontinuierlich, gegebenenfalls unter vermindertem Druck, z.B. über eine Kolonne abdestilliert und können direkt wieder der Umesterung (R1) zugeführt werden.

Als hochsiedende Alkohole kommen z. B. Glycerin, Rückstände der 2-Ethylhexanolherstellung, Rückstände oder Nebenfraktionen der Polyethylenglykol oder Trimethylolpropanherstellung. Der Vorteil besteht u. a. darin, daß wertvolle Alkohole zurückgewonnen werden (Erhöhung der Ausbeute) und die Rückstände, die vorteilhaft verbrannt werden, besser handhabbar sind. Durch gezielte Wahl der Art und der Menge des hochsiedenden Alkohols kann nämlich die Viskosität des Rückstandes in weiten Grenzen variiert werden.

Vorteilhaft kann auch das Destillat der Katalysatorherstellung aus Stufe "1" zur Einstellung der Viskosität, z.B. durch Verdünnung, verwendet werden.

### 10. Azeotropverwertung

Das ausgeschleuste Gemisch aus niederem Alkanol und dem entsprechendem (Meth)acrylsäureester aus Stufe "2" beziehungsweise "3" kann direkt, d. h. ohne eine vorherige Reinigung, in Gegenwart von starken Säuren, z.B. Schwefelsäure oder Sulfonsäure, mit (Meth)acrylsäure auf bekannte Weise zur Reaktion gebracht werden (Kirk Othmer, Encyclopedia of Chemical Technology, 4^{th} Ed., 1994, Seiten 301 - 302). Das niedere Alkanol wird dabei in den entsprechenden (Meth)acrylsäureester umgewandelt, der wieder als Ausgangsmaterial für die erfindungsgemäße Umesterung eingesetzt werden kann.

Eine weitere bevorzugte Ausführungsform besteht darin, daß das Gemisch nicht dem Veresterungsreaktor sondern zumindest z.T. der Aufarbeitung einer Anlage zur Herstellung des niederen (Meth)acrylsäureesters zugeführt wird. Vorzugsweise wird das Gemisch der Waschkolonne zugeführt, in der die Trennung des Alkanols vom Zielester erfolgt, wobei das Alkanol aus der wäßrigen Phase zurückgewonnen und bei der Veresterung wiederverwendet wird (ibid. S 301).

Aufgrund der Verwendung des speziellen Stabilisatorsystems in den einzelnen Aufarbeitungsstufen des Umesterungsgemisches, wird die Bildung von Polymerisat und Ablagerungen weitgehend verhindert. Es müssen daher keine zusätzlichen speziellen Bedingungen in den verschiedenen Destillationsschritten eingehalten werden.

Die Laufzeit der Anlage beträgt unter den geschilderten Bedingungen im allgemeinen mindestens 150 Tage.

Der Gehalt an Ethylenglykoldi(meth)acrylat und vinyloxyethyl(meth)acrylat in den erfindungsgemäß hergestellten Dialkylaminoethyl(meth)acrylaten beträgt im allgemeinen nicht mehr als 100 ppm, bevorzugt nicht mehr als 50 ppm, besonders bevorzugt nicht mehr als 20 ppm und insbesondere nicht mehr als 10 ppm.

Die erfindungsgemäß hergestellten Dialkylaminoalkyl(meth)acrylate, besonders Dialkylaminoethyl(meth)acrylate und speziell Dimethylaminoethyl(meth)acrylate sind wertvolle Monomere für die Herstellung von Copolymerisaten. Als Monomere werden sie in der vorliegenden Form oder nach Quaternisierung in die Polymerisation eingesetzt.

Übliche Quaternisierungsmittel sind beispielsweise Benzylhalogenide wie z.B. Benzylchlorid, Alkylhalogenide wie z.B. Methylchlorid, Ethylchlorid, Methylbromid, Ethylendichlorid oder Allylchlorid, Alkylenoxide wie z.B. Ethylenoxid, Propylenoxid, Styroloxid, *iso*-Butylenoxid oder Vinyloxiran, bevorzugt Ethylenoxid oder Propylenoxid und besonders bevorzugt Ethylenoxid, Alkylphosphite oder -phosphonate wie z.B. Trimethylphosphit oder Triethylphosphit, Dialkylsulfate wie z.B. Dimethylsulfat oder Diethylsulfat, Dialkylcarbonate wie z.B. Dimethylcarbonat, Diethylcarbonat oder Di-n-butylcarbonat, Chlorhydrin oder Epichlorhydrin.

Besonders solche Copolymere, die quaternisierte Monomere einpolymerisiert enthalten, finden Verwendung in der Wasseraufbereitung, beispielsweise als Ionentauscherharze oder als Bestandteil von Membranen.

Das erfindungsgemäße Verfahren wird durch das nachfolgende Beispiel näher erläutert, ohne es darauf einzuschränken.

In dieser Schrift verwendete ppm- und Prozentangaben beziehen sich, falls nicht anders angegeben, auf Gewichtsprozente und -ppm.

### Beispiel 1 (Herstellung des Katalysators)

In einem Rührreaktor mit Wandbeheizung und aufgesetzter Füllkörperkolonne wurde ein Gemisch aus 1300 Teilen Dimethylaminoethanol und 400 Teilen Tetraisopropyltitanat auf 100 °C erhitzt und das gebildete Isopropanol kontinuierlich über die Kolonne ausgeschleust (Kopf temperatur 70 °C, 300 mbar). Innerhalb von 4 Stunden wurden 250 Teile Destillat gewonnen, der Rückstand enthielt 4,6 Gew% Titan, enthielt höchstens 0,1 Gew% *iso*-Propanol und wurde direkt als Umesterungskatalysator eingesetzt.

### Beispiel 2 (Herstellung Dimethylaminoethylacrylat)

Im kontinuierlichen Betrieb wurden stündlich 266 Teile Dimethylaminoethanol mit einem Gehalt an Ethylenglycol und Vinyloxyethanol von jeweils weniger als 10 ppm, 780 Teile eines Gemisches aus Methylacrylat und dem Destillat der Methylacrylat-Abtrennung, 17 Teile Katalysatorlösung aus Beispiel 1 und 69 Teile Rückkatalysator (Sumpf der Katalysatorabtrennung) dem ersten Reaktor einer aus zwei Reaktoren bestehenden Reaktorkaskade zugeführt. Die Reaktoren waren jeweils mit einer aufgesetzten Füllkörperkolonne und zwei seriell angeordneten Kondensatoren ausgerüstet. Die Wärmezufuhr erfolgte über außenliegende Wärmetauscher. Außerdem wurden 270 Teile des Kondensats der Leichtsiederabtrennung stündlich über einen Zulauf in der Mitte der Kolonne des ersten Reaktors zugegeben. Die Reaktionstemperatur betrug 100° C. Das bei der Umesterung entstehende Methanol wurde als Gemisch mit Methylacrylat am Kopf der Reaktorkolonnen (63° C) ausgeschleust und kondensiert. Die vereinigten Kondensate wurden teilweise als Rücklauf in die Kolonnen zurückgeführt (jeweils ca. 2100 Teile) und der Rest (280 Teile) ausgeschleust und für eine Verwendung in der Methylacrylatherstellung gesammelt. Der Gehalt an Dimethylaminoethanol betrug weniger als 0,05 %.

Die Stabilisierung der Kondensate erfolgt durch Zugabe von 56 Teilen einer Lösung von 0,5 % Phenothiazin und 0,05 % 4-Hydroxy-2,2,6,6-tetramethylpiperidin-N-Oxyl in Methylacrylat auf den ersten Kondensator der Kolonne des ersten Reaktors und von 115 Teilen dieser Lösung auf den ersten Kondensator der Kolonne des zweiten Reaktors. Die vereinigten Kondensate enthielten ca. 36 Gew.% Methanol und ca. 64 Gew.% Methylacrylat.

Der Austrag des zweiten Reaktors (1300 Teile) wurde einem isolierten Behälter zugeführt, dessen durchschnittliche Temperatur bei 88 °C lag. Der Austrag dieses Behälters wurde teilweise wieder dem Reaktor R2 und teilweise (1 : 1) einer Füllkörperkolonne zugeführt (Zulauf oberhalb der Kolonnenmitte, 1300 Teile), wo er in ein Kopfprodukt (Kopftemperatur 56° C, 300 mbar) und ein katalysatorhaltiges Sumpfprodukt (Sumpf temperatur 130 °C), das im wesentlichen aus Dimethylaminoethylacrylat und Dimethylaminoethanol bestand, aufgetrennt wurde.

Das Produkt wurde durch Einsprühen von 95 Teilen der oben beschriebenen Stabilisatorlösung in den Kondensatorkopf stabilisiert. Das Kondensat wurde mit 170 Teilen Methylacrylat vermischt und teilweise (98 Teile/h) als Rücklauf wieder der Kolonne und der Rest (780 Teile/h) dem ersten Reaktor zugeführt. Er bestand im wesentlichen aus 96,5 % Methylacrylat, 1,5 % Dimethylaminoethylacrylat und 1,3 % Methanol.

Das Sumpfprodukt wurde einer Flash-Verdampfung (130 °C, 65 mbar) unterzogen, wobei ein Destillat gewonnen wurde, das mit 0,1 % Hydrochinonmonomethylether stabilisiert wurde und ca. 90 % Dimethylaminoethylacrylat enthielt. Das anfallende katalysatorhaltige Sumpfprodukt wurde zum Teil wieder dem ersten Reaktor (69 Teile/h) und der Rest (30 Teile/h) der Rückstandsbehandlung zugeführt.

Das Destillat (685 Teile) wurde mit dem Destillat der Rückstandsbehandlung (137 Teile) in einer weiteren Füllkörperkolonne (Zulauf unterhalb der Kolonnenmitte) in eine Leichtsiederfraktion (Kopftemperatur 73° C, 60 mbar) und ein Sumpfprodukt, das im wesentlichen aus Dimethylaminoethylacrylat (97,8 %) und Michael-Additionsprodukt (1,9 %) bestand, aufgetrennt. Die Leichtsiederfraktion wurde durch Einsprühen von 19 Teile/h der beschriebenen Stabilisatorlösung in den Kopf des Kondensators stabilisiert und bestand im wesentlichen aus 50,6 % Dimethylaminoethylacrylat, 29,6 % Dimethylaminoethanol und 21,9 % Methylacrylat. 270 Teile der Leichtsiederfraktion wurden über die Kolonne dem 1. Reaktor zugeführt, der Rest wurde als Rücklauf am Kopf der Leichtsiederkolonne aufgebracht. Aus dem Sumpfprodukt wurde in einem weiteren Destillationsschritt (Füllkörperkolonne, Zulauf oberhalb des 1. Drittels der Kolonne) Dimethylaminoethylacrylat als Kopfprodukt (85° C, 60 mbar) isoliert. Das Destillat wurde mit 0,1 Teilen Hydrochinonmonomethylether als 1 gewichtsprozentige Lösung in Reinprodukt stabilisiert, 380 Teile davon als Rücklauf in den Kolonnenkopf zurückgeführt und 445 Teile als Reinprodukt abgeführt. Die Reinheit betrug 99,9 %, die Ausbeute 98 % bezüglich Dimethylaminoethanol, der Gehalt an Ethylenglycoldiacrylat und Vinyloxyethylacrylat jeweils weniger als 10 ppm.

Der Rückstand der Reindestillation und der ausgeschleuste Rückstand der Katalysatorabtrennung wurden einem Reaktor mit aufgesetzter Füllkörperkolonne zugeführt und bei 140° C das restliche Dimethylaminoethylacrylat abdestilliert und gleichzeitig die Michael-Additionsprodukte zurückgespalten. Es wurden 137 Teile/h Destillat isoliert und dem Zulauf zur Leichtsiederdestillation zugegeben. Der Rückstand enthielt keinen Feststoff und war pumpfähig.

### Beispiel 3 (Rückstandsaufarbeitung)

In einem Reaktor wurde der Rückstand der Rückstandsbehandlung mit Glycerin 1:1 vermischt und auf 160° C erhitzt. Das freigesetzte Dimethylaminoethanol wurde kontinuierlich gasförmig ausgeschleust und kondensiert. Es wurden aus 100 Teilen Rückstand 37 Teile eines Destillats gewonnen, das im wesentlichen aus Dimethylaminoethanol bestand.

### Beispiel 4

Im kontinuierlichen Betrieb wurden stündlich 680 Teile Dimethylaminoethanol, 2016 Teile eines Gemisches aus Ethylacrylat und dem Destillat der Ethylacrylat-Abtrennung, 70 Teile Katalysatorlösung aus Beispiel 1 und 110 Teile Rückkatalysator (Sumpf der Katalysatorabtrennung) dem ersten Reaktor einer aus zwei Reaktoren bestehenden Reaktorkaskade zugeführt. Die Reaktoren waren jeweils mit einer aufgesetzten Füllkörperkolonne und einem Kondensator ausgerüstet. Die Wärmezufuhr erfolgte über außenliegende Wärmetauscher. Außerdem wurden 236 Teile des Kondensats der Leichtsiederabtrennung stündlich über einen Zulauf in der Mitte der Kolonne des ersten Reaktors zugegeben. Der Austrag des zweiten Reaktors wurde einem mit einem Umlaufverdampfer ausgerüsteten Behälter zugeführt, der gasseitig mit der Kolonne des zweiten Reaktors verbunden war. Die Reaktionstemperaturen betrugen 110 beziehungsweise 115 ° C, im Behälter 119 °C. Das bei der Umesterung entstehende Ethanol wurde als Gemisch mit Ethylacrylat (48 % Ethanol) am Kopf der Reaktorkolonnen ausgeschleust und kondensiert. Die vereinigten Kondensate wurden teilweise als Rücklauf in die Kolonnen zurückgeführt (jeweils ca. 2100 Teile) und der Rest (806 Teile) ausgeschleust und für eine Verwendung in der Ethylacrylatherstellung gesammelt. Die Stabilisierung der Kondensate erfolgt durch Zugabe von 120 Teilen einer Lösung von 0,5 % Phenothiazin und 0,05 % 4-Hydroxy-2,2,6,6-tetramethylpiperidin-N-Oxyl in Ethylacrylat auf jeden Kondensator. Die vereinigten Kondensate enthielten ca. 48 Gew.% Ethanol und ca. 52 Gew.% Ethylacrylat, der Gehalt an Dimethylaminoethanol betrug weniger als 0,1 %.

Der Austrag des Behälters wurde einer Füllkörperkolonne zugeführt (Zulauf in der Kolonnenmitte), wo er in ein Kopfprodukt (Kopftemperatur 88° C, 500 mbar) und ein katalysatorhaltiges Sumpfprodukt (Sumpftemperatur 140 °C) aufgetrennt wurde.

Das Kopfprodukt wurde durch Einsprühen von 110 Teilen der oben beschriebenen Stabilisatorlösung in den Kondensatorkopf stabilisiert. Das Kondensat wurde mit 1170 Teilen Ethylacrylat vermischt und teilweise (380 Teile/h) als Rücklauf wieder der Kolonne und der Rest (2016 Teile/h) dem ersten Reaktor zugeführt.

Das Sumpfprodukt wurde einem Flashverdampfer zugeführt, der zusätzlich mit einem Umlaufverdampfer ausgerüstet war (135 °C, 80 mbar). Das Destillat wurde mit 50 Teilen Stabilisatorlösung (s.o.) stabilisiert und enthielt ca. 90 % Dimethylaminoethylacrylat. Das Sumpfprodukt wurde teilweise (110 Teile) dem ersten Reaktor zugeführt und der Rest (70 Teile/h) der Rückstandsbehandlung zugeführt.

Das Verhältnis der Michael-Additionsproduktbildung (EP-A 906 902, Seite 9) war, über beide Destillationsstufen berechnet, negativ (-1,3%), das bedeutet eine teilweise Reduzierung (Rückspaltung) der Addukte unter den angegebenen Bedingungen.

Die weitere, analog Beispiel 2 durchgeführte Aufarbeitung des Destillats der Flashverdampfung lieferte 1189 Teile Dimethylaminoethylacrylat in einer Reinheit von 99,9 %, was einer Ausbeute von 97,9 % bezüglich Dimethylaminoethanol entspricht.

### Vergleichsbeispiel

Es wurde analog Beispiel 4 verfahren, die Sumpftemperatur bei der Ethylacrylatabtrennung betrug jedoch 110 °C und die Temperatur bei der Katalysatorabtrennung (Flashverdampfer) 110 °C.

Das auf Basis der gaschromatographischen Analysen der einzelnen Ströme ermittelte Verhältnis der Michael-Additionsproduktbildung betrug +0,1%, d.h. es erfolgte eine geringe Zunahme an Michael-Additionsprodukt.

## Patentansprüche

1. Verfahren zur Herstellung von (Meth)acrylsäureestern IV durch Umesterung eines (Meth)acrylsäureesters I mit einem Alkohol R²OH, der mindestens ein Kohlenstoffatom mehr aufweist als die Alkoholkomponente (R¹O-) im umzuesternden (Meth)acrylsäureester I, in Gegenwart eines Katalysators oder Katalysatorgemisches, **dadurch gekennzeichnet, daß** der bei der Umesterung freigesetzte niedere Alkanol R¹OH gemeinsam mit einem Teil des niederen (Meth)acrylsäureesters I abgetrennt und ohne einen zusätzlichen Reinigungsschritt in eine Anlage zur Herstellung und/oder Aufarbeitung des (Meth)acrylsäureesters I eingespeist wird und aus dem Reaktionsaustrag der Umesterung
entweder zunächst die Hauptmenge des (Meth)acrylsäureesters I abgetrennt und dann destillativ vom verwendeten Katalysator abgetrennt wird (Katalysatorabtrennung),
oder zunächst destillativ vom verwendeten Katalysator abgetrennt (Katalysatorabtrennung) und dann die Hauptmenge des (Meth)acrylsäureesters I abgetrennt wird
und dann von dem erhaltenen Gemisch leichter als der (Meth)acrylsäureester IV siedende Komponenten destillativ im wesentlichen abgetrennt werden (Leichtsiederabtrennung) und anschließend der (Meth)acrylsäureester IV reindestilliert wird (Reindestillation),
wobei man die Katalysatorabtrennung bei Temperaturen von 125 bis 160°C durchführt.

2. Verfahren zur Herstellung von (Meth)acrylsäureestern IV durch Umesterung eines (Meth)acrylsäureesters I mit einem Alkohol R²OH, der ausgewählt ist aus n-Propanol, *iso*-Propanol, Allylalkohol, n-Butanol, 1-Methyl-propanol, 2-Methyl-propanol, tert.-Butanol, n-Pentanol, 1-Methyl butanol, 2-Methyl-butanol, 3-Methyl-butanol, 2,2-Dimethylpropanol, n-Hexanol, 1-Methyl-pentanol, 2-Methyl-pentanol, 3-Methyl-pentanol, 4-Methyl-pentanol, 1,1-Dimethyl-butanol, 2,2-Dimethyl-butanol, 3,3-Dimethyl-butanol, 1,2-Dimethyl-butanol, n-Heptanol, 1-Methyl-hexanol, 2-Methyl-hexanol, 3-Methyl-hexanol, 4-Methylhexanol, 1,2-Dimethyl-pentanol, 1,3-Dimethyl-pentanol, 1,1-Dimethyl-pentanol, 1,1,2,2-Tetramethyl-propanol, Benzylalkohol, n-Octanol, 2-Ethylhexanol, n-Nonanol, 1-Methyl-octanol, 2-Methyl-octanol, n-Decanol, n-Undecanol, 1-Methyl-decanol, 2-Methyl-decanol, n-Dodecanol, 2,4-Diethyl-octanol, Cyclopentanol, Cyclohexanol, 4-*tert*.-Butyl-cyclohexanol, Cycloheptanol, Cyclododecanol, 2-(Dimethylamino)-ethanol, 3-(Dimethylamino)-propanol, 4-(Dimethylamino)-butanol, 5-(Dimethylamino)-pentanol, 6-(Dimethylamino)-hexanol, 8-(Dimethylamino)-octanol, 10-(Dimethylamino)-decanol, 12-(Dimethylamino)-dodecanol, 2-(Diethylamino)-ethanol, 3-(Diethylamino)-propanol, 4-(Diethylamino)-butanol, 5-(Diethylamino)-pentanol, 6-(Diethylamino)-hexanol, 8-(Diethylamino)-octanol, 10-(Diethylamino)-decanol, 12-(Diethylamino)-dodecanol, 2-(Di-(iso-propyl)-amino)-ethanol, 3-(Di-(iso-propyl)-amino)-propanol, 4-(Di-(iso-propyl)-amino)-butanol, 5-(Di-(iso-propyl)-amino)-pentanol, 6-(Di-(iso-propyl)-amino)-hexanol, 8-(Di-(iso-propyl)-amino)-octanol, 10-(Di-(iso-propyl)-amino)-decanol, 12-(Di-(iso-propyl)-amino)-dodecanol, 2-(Dibutylamino)-ethanol, 3-(Dibutylamino)-propanol, 4-(Dibutylamino)-butanol, 5-(Dibutylamino)-pentanol, 6-(Dibutylamino)-hexanol, 8-(Dibutylamino)-octanol, 10-(Dibutylamino)-decanol, 12-(Dibutylamino)-dodecanol, 2-(Dihexylamino)-ethanol, 3-(Dihexylamino)-propanol, 4-(Dihexylamino)-butanol, 5-(Dihexylamino)-pentanol, 6-(Dihexylamino)-hexanol, 8-(Dihexylamino)-octanol, 10-(Dihexylamino)-decanol, 12-(Dihexylamino)-dodecanol, 2-(Methyl-ethyl-amino)-ethanol, 2-(Methyl-propylamino)-ethanol, 2-(Methyl-iso-propyl-amino)-ethanol, 2-(Methyl-butyl-amino)-ethanol, 2-(Methyl-hexyl-amino)-ethanol, 2-(Methyl-octyl-amino)-ethanol, 2-(Ethyl-propylamino)-ethanol, 2-(Ethyl-iso-propyl-amino)-ethanol, 2-(Ethylbutyl-amino)-ethanol, 2-(Ethyl-hexyl-amino)-ethanol, 2-(Ethyl-octyl-amino)-ethanol, 3-(Methyl-ethylamino)-propanol, 3-(Methyl-propyl-amino)-propanol, 3-(Methyliso-propyl-amino)-propanol, 3-(Methyl-butyl-amino)-propanol, 3-(Methyl-hexyl-amino)-propanol, 3-(Methyl-octylamino)-propanol, 3-(Ethyl-propyl-amino)-propanol, 3-(Ethyliso-propyl-amino)-propanol, 3-(Ethyl-butyl-amino)-propanol, 3-(Ethyl-hexyl-amino)-propanol, 3-(Ethyl-octylamino)-propanol, 4-(Methyl-ethyl-amino)-butanol, 4-(Methyl-propyl-amino)-butanol, 4-(Methyl-iso-propyl-amino)-butanol, 4-(Methyl-butyl-amino)-butanol, 4-(Methyl-hexylamino)-butanol, 4-(Methyl-octyl-amino)-butanol, 4-(Ethyl-propyl-amino)-butanol, 4-(Ethyl-iso-propyl-amino)-butanol, 4-(Ethyl-butyl-amino)-butanol, 4-(Ethyl-hexyl-amino)-butanol, 4-(Ethyl-octyl-amino)-butanol, 2-(N-Piperidinyl)-ethanol, 3-(N-Piperidinyl)-propanol, 4-(N-Piperidinyl)-butanol, 5-(N-Piperidinyl)-pentanol, 6-(N-Piperidinyl)-hexanol, 8-(N-Piperidinyl)-octanol, 10-(N-Piperidinyl)-decanol, 12-(N-Piperidinyl)-dodecanol, 2-(N-Pyrrolidinyl)-ethanol, 3-(N-Pyrrolidinyl)-propanol, 4-(N-Pyrrolidinyl)-butanol, 5-(N-Pyrrolidinyl)-pentanol, 6-(N-Pyrrolidinyl)-hexanol, 8-(N-Pyrrolidinyl)-octanol, 10-(N-Pyrrolidinyl)-decanol, 12-(N-Pyrrolidinyl)-dodecanol, 2-(N-Morpholino)-ethanol, 3-(N-Morpholino)-propanol, 4-(N-Morpholino)-butanol, 5-(N-Morpholino)-pentanol, 6-(N-Morpholino)-hexanol, 8-(N-Morpholino)-octanol, 10-(N-Morpholino)-decanol, 12-(N-Morpholino)-dodecanol, 2-(N'-Methyl-N-Piperazinyl)-ethanol, 3-(N'-Methyl-N-Piperazinyl)-propanol, 4-(N'-Methyl-N-Piperazinyl)-butanol, 5-(N'-Methyl-N-Piperazinyl)-pentanol, 6-(N'-Methyl-N-Piperazinyl)-hexanol, 8-(N'-Methyl-N-Piperazinyl)-octanol, 10-(N'-Methyl-N-Piperazinyl)-decanol, 12-(N'-Methyl-N-Piperazinyl)-dodecanol, 2-(N'-Ethyl-N-Piperazinyl)-ethanol, 3-(N'-Ethyl-N-Piperazinyl)-propanol, 4-(N'-Ethyl-N-Piperazinyl)-butanol, 5-(N'-Ethyl-N-Piperazinyl)-pentanol, 6-(N'-Ethyl-N-Piperazinyl)-hexanol, 8-(N'-Ethyl-N-Piperazinyl)-octanol, 10-(N'-Ethyl-N-Piperazinyl)-decanol, 12-(N'-Ethyl-N-Piperazinyl)-dodecanol, 2-(N'-*iso*-Propyl-N-Piperazinyl)-ethanol, 3-(N'-*iso*-Propyl-N-Piperazinyl)-propanol, 4-(N'-*iso*-Propyl-N-Piperazinyl)-butanol, 5-(N'-*iso*-Propyl-N-Piperazinyl)-pentanol, 6-(N'-*iso*-Propyl-N-Piperazinyl)-hexanol, 8-(N'-*iso*-Propyl-N-Piperazinyl)-octanol, 10-(N'-*iso*-Propyl-N-Piperazinyl)-decanol, 12-(N'-*iso*-Propyl-N-Piperazinyl)-dodecanol, 3-Oxa-butanol, 3-Oxa-pentanol, 2,2-Dimethyl-4-oxa-pentanol, 3,6-Dioxa-heptanol, 3,6-Dioxaoctanol, 3,6,9-Trioxa-decanol, 3,6,9-Trioxa-undecanol, 4-Oxapentanol, 4-Oxa-hexanol, 4-Oxa-heptanol, 4,8-Dioxa-nonanol, 4,8-Dioxa-decanol, 4,8-Dioxa-undecanol, 5-Oxa-hexanol oder 5,10-Dioxa-undecanol oder ethoxylierte und/oder propoxylierte Alkohole sowie gemischt-ethoxylierte/propoxylierte Alkohole wie R⁵-(O-CH₂-CH₂)ₓ-OH oder R⁵-(O-CH(CH₃)-CH₂)ₓ-OH beziehungsweise R⁵-(O-CH₂-CH(CH₃))ₓ-OH, worin R⁵ für C₁ bis C₂₀-Alkyl und x für eine ganze Zahl zwischen 1 und 20 steht, oder um ethoxylierte und/oder propoxylierte Aminoalkohole R³₂N(-CH₂CH₂-O)_{y}-H oder R³₂N(-CH(CH₃)-CH₂-O)_{y}-H beziehungsweise R³₂N(-CH₂-CH(CH₃)-O-)_{y}-H, worin y für eine ganze Zahl zwischen 1 und 4 steht, in Gegenwart eines Katalysators oder Katalysatorgemisches, **dadurch gekennzeichnet, daß** der bei der Umesterung freigesetzte niedere Alkanol R¹OH, der ausgewählt ist aus Methanol, Ethanol, n-Propanol, iso-Propanol, n-Butanol, iso-Butanol, 2-Butanol und tert.-Butanol, gemeinsam mit einem Teil des niederen (Meth)acrylsäureesters I abgetrennt und ohne einen zusätzlichen Reinigungsschritt in eine Anlage zur Herstellung und/oder Aufarbeitung des (Meth)acrylsäureesters I eingespeist wird und aus dem Reaktionsaustrag der Umesterung
entweder zunächst die Hauptmenge des (Meth)acrylsäureesters I abgetrennt und dann destillativ vom verwendeten Katalysator abgetrennt wird (Katalysatorabtrennung),
oder zunächst destillativ vom verwendeten Katalysator abgetrennt (Katalysatorabtrennung) und dann die Hauptmenge des (Meth)acrylsäureesters I abgetrennt wird
und dann von dem erhaltenen Gemisch leichter als der (Meth)acrylsäureester IV siedende Komponenten destillativ im wesentlichen abgetrennt werden (Leichtsiederabtrennung) und anschließend der (Meth)acrylsäureester IV reindestilliert wird (Reindestillation),
wobei man die Katalysatorabtrennung bei Temperaturen von 125 bis 160°C durchführt.

3. Verfahren nach Anspruch 2, **dadurch gekennzeichnet**, der Alkohol R²OH ausgewählt ist aus Dimethylaminoethanol, Diethylaminoethanol, Di-n-butylaminoethanol, 3-Dimethylaminopropanol, 3-Diethylaminopropanol und 3-Di-n-butylaminopropanol.

4. Verfahren nach einem der vorstehenden Ansprüche, **dadurch gekennzeichnet, daß** man die Abtrennung des (Meth)acrylsäureesters I bei Temperaturen von 120 bis 160 °C durchführt.

5. Verfahren nach einem der vorstehenden Ansprüche, **dadurch gekennzeichnet, daß** der Siedepunkt des Alkohols R²OH 20 °C oder mehr über dem des Alkohols R¹OH liegt.

6. Verfahren nach einem der vorstehenden Ansprüche, **dadurch gekennzeichnet, daß** das der dem Reaktor aufgesetzten Kolonne/den Reaktoren aufgesetzten Kolonnen entnommene Destillat
im Fall von Methanolat als Alkoholkomponente (R¹O-) im umzuesternden (Meth)acrylsäureester I 20 - 40 Gew. % Methanol und
im Fall von Ethanolat als Alkoholkomponente (R¹O-) im umzuesternden (Meth)acrylsäureester I 30 - 65 Gew. % Ethanol enthält.

7. Verfahren nach einem der vorstehenden Ansprüche, **dadurch gekennzeichnet, daß** der Rückstand der Katalysatorabtrennung zumindest teilweise in die Umesterung rückgeführt wird.

8. Verfahren nach einem der vorstehenden Ansprüche, **dadurch gekennzeichnet, daß** der Rückstand der Katalysatorabtrennung getrennt oder gemeinsam mit dem Rückstand der Reindestillation zumindest teilweise einer thermischen und/oder katalytischen Behandlung unterworfen wird.

9. Verfahren nach Anspruch 7 oder 8, **dadurch gekennzeichnet, daß** ein der thermischen und/oder katalytischen Behandlung entnommenes Destillat in die Leichtsiederabtrennung und/oder die Umesterung rückgeführt wird.

10. Verfahren nach einem der Ansprüche 7 bis 9, **dadurch gekennzeichnet, daß** der Sumpfaustrag der thermischen und/oder katalytischen Behandlung mit einer hochsiedenden, Hydroxylgruppen enthaltenden Substanz umgesetzt wird und die dabei abdestillierten Leichtsieder in die Umesterung rückgeführt werden.

11. Verfahren nach einem der vorstehenden Ansprüche, **dadurch gekennzeichnet, daß** zumindest ein Teil des Austrags der Umesterung aus einem isolierten Zwischenbehälter wieder in die Umesterung zurückgeführt wird.

12. Verfahren nach Anspruch 11, **dadurch gekennzeichnet, daß** der isolierte Zwischenbehälter beheizt ist.

13. Verfahren nach Anspruch 11 oder 12, **dadurch gekennzeichnet, daß** der isolierte Zwischenbehälter mit dem letzten Reaktor der Umesterung und/oder der diesem aufgesetzten Kolonne in Verbindung steht.

14. Verfahren nach einem der vorstehenden Ansprüche, **dadurch gekennzeichnet, daß** als Katalysator Titanalkoholat Ti(OR²)₄ eingesetzt wird.

15. Verfahren nach Anspruch 14, **dadurch gekennzeichnet, daß** das Titanalkoholat Ti(OR²)₄ aus einem niederen Titanalkoholat Ti(OR⁴)₄, wobei R⁴ für C₁ - C₈-Alkyl steht, durch Umsetzung mit einem höheren Alkohol R²OH erhalten wird, wobei R² die obige Bedeutung hat und R²OH und R⁴OH über ihre Siedepunkte Kp die Bedingung
Kp. (R²OH) ≥ Kp. (R⁴OH) + 20 °C
erfüllen.

16. Verfahren nach Anspruch 15, **dadurch gekennzeichnet, daß** das bei der Umsetzung entstehende Alkanol R⁴OH aus der Umsetzung entfernt wird.

17. Verfahren nach Anspruch 15 oder 16, **dadurch gekennzeichnet, daß** das erhaltene Reaktionsgemisch weniger als 400 ppm R⁴OH enthält.

18. Verfahren nach einem der vorstehenden Ansprüche, **dadurch gekennzeichnet, daß** es zumindest teilweise in Gegenwart mindestens eines der folgenden Stabilisatoren ausgeführt wird: Phenothiazin, 4-Methyl-2,6-*tert*.-Butylphenol, Hydrochinonmonomethylether, 4-Hydroxy-2,2,6,6-tetramethylpiperidin-N-Oxyl, 4-Oxo-2,2,6,6-tetramethylpiperidin-N-Oxyl.

19. Verfahren nach Anspruch 3, **dadurch gekennzeichnet, daß** das eingesetzte Dialkylaminoethanol einen Gehalt an Vinyloxyethanol von nicht mehr als 100 ppm aufweist.

20. Verfahren nach Anspruch 3, **dadurch gekennzeichnet, daß** das eingesetzte Dialkylaminoethanol einen Gehalt an Ethylenglykol von nicht mehr als 100 ppm aufweist.

21. Verfahren nach einem der vorstehenden Ansprüche, **dadurch gekennzeichnet, daß** R² 2-(Dimethylamino)-ethyl-, 2-(Di-n-butylamino)-ethyl- oder 2-(Diethylamino)-ethyl- und R¹ Methyloder Ethyl- ist.

## Claims

1. A process for the preparation of (meth)acrylates IV by transesterification of a (meth)acrylate I with an alcohol R²OH which has at least one carbon atom more than the alcohol component (R¹O-) in the (meth)acrylate I to be transesterified, in the presence of a catalyst or catalyst mixture, wherein the lower alkanol R¹OH liberated in the transesterification is separated off together with a part of the lower (meth)acrylate I and is fed, without an additional purification step, into a plant for the production and/or working-up of the (meth)acrylate I and, from the discharge of the transesterification reaction,
either first the major amount of the (meth)acrylate I is separated off and is then separated by distillation from the catalyst used (catalyst removal),
or is first separated by distillation from the catalyst used (catalyst removal) and then the major amount of the (meth)acrylate I is separated off
and then components having a lower boiling point than the (meth)acrylate IV are substantially separated from the resulting mixture by distillation (low boiler removal) and then the (meth)acrylate IV is purified by distillation (distillative purification),
the catalyst removal being carried out at from 125 to 160°C.

2. A process for the preparation of (meth)acrylates IV by transesterification of a (meth)acrylate I with an alcohol R²OH which is selected from n-propanol, isopropanol, allyl alcohol, n-butanol, 1-methylpropanol, 2-methylpropanol, tert-butanol, n-pentanol, 1-methylbutanol, 2-methylbutanol, 3-methylbutanol, 2,2-dimethylpropanol, n-hexanol, 1-methylpentanol, 2-methylpentanol, 3-methylpentanol, 4-methylpentanol, 1,1-dimethylbutanol, 2,2-dimethylbutanol, 3,3-dimethylbutanol, 1,2-dimethylbutanol, n-heptanol, 1-methylhexanol, 2-methylhexanol, 3-methylhexanol, 4-methylhexanol, 1,2-dimethylpentanol, 1,3-dimethylpentanol, 1,1-dimethylpentanol, 1,1,2,2-tetramethylpropanol, benzyl alcohol, n-octanol, 2-ethylhexanol, n-nonanol, 1-methyloctanol, 2-methyloctanol, n-decanol, n-undecanol, 1-methyldecanol, 2-methyldecanol, n-dodecanol, 2,4-diethyloctanol, cyclopentanol, cyclohexanol, 4-*tert*-butylcyclohexanol, cycloheptanol, cyclododecanol, 2-(dimethylamino)ethanol, 3-(dimethylamino)propanol, 4-(dimethylamino)butanol, 5-(dimethylamino)pentanol, 6-(dimethylamino)hexanol, 8-(dimethylamino)octanol, 10-(dimethylamino)decanol, 12-(dimethylamino)dodecanol, 2-(diethylamino)ethanol, 3-(diethylamino)propanol, 4-(diethylamino)butanol, 5-(diethylamino)pentanol, 6-(diethylamino)hexanol, 8-(diethylamino)octanol, 10-(diethylamino)decanol, 12-(diethylamino)dodecanol, 2-(di(isopropyl)amino)ethanol, 3-(di(isopropyl)-amino)propanol, 4-(di(isopropyl)amino)butanol, 5-(di(isopropyl)amino)pentanol, 6-(di(isopropyl)amino)-hexanol, 8-(di(isopropyl)amino)octanol, 10-(di(isopropyl)-amino)decanol, 12-(di(isopropyl)amino)dodecanol, 2-(dibutylamino)ethanol, 3-(dibutylamino)propanol, 4-(dibutylamino)butanol, 5-(dibutylamino)pentanol, 6-(dibutylamino)hexanol, 8-(dibutylamino)octanol, 10-(dibutylamino)decanol, 12-(dibutylamino)dodecanol, 2-(dihexylamino)ethanol, 3-(dihexylamino)propanol, 4-(dihexylamino)butanol, 5-(dihexylamino)pentanol, 6-(dihexylamino)hexanol, 8-(dihexylamino)octanol, 10-(dihexylamino)decanol, 12-(dihexylamino)dodecanol, 2-(methylethylamino)ethanol, 2-(methylpropylamino)ethanol, 2-(methylisopropylamino)ethanol, 2-(methylbutylamino)ethanol, 2-(methylhexylamino)ethanol, 2-(methyloctylamino)ethanol, 2-(ethylpropylamino)ethanol, 2-(ethylisopropylamino)ethanol, 2-(ethylbutylamino)ethanol, 2-(ethylhexylamino)ethanol, 2-(ethyloctylamino)ethanol, 3-(methylethylamino)propanol, 3-(methylpropylamino)propanol, 3-(methylisopropylamino)propanol, 3-(methylbutylamino)propanol, 3-(methylhexylamino)propanol, 3-(methyloctylamino)propanol, 3-(ethylpropylamino)propanol, 3-(ethylisopropylamino)propanol, 3-(ethylbutylamino)propanol, 3-(ethylhexylamino)propanol, 3-(ethyloctylamino)propanol, 4-(methylethylamino)butanol, 4-(methylpropylamino)butanol, 4-(methylisopropylamino)butanol, 4-(methylbutylamino)butanol, 4-(methylhexylamino)butanol, 4-(methyloctylamino)butanol, 4-(ethylpropylamino)butanol, 4-(ethylisopropylamino)butanol, 4-(ethylbutylamino)butanol, 4-(ethylhexylamino)butanol, 4-(ethyloctylamino)butanol, 2-(N-piperidinyl)ethanol, 3-(N-piperidinyl)propanol, 4-(N-piperidinyl)butanol, 5-(N-piperidinyl)pentanol, 6-(N-piperidinyl)hexanol, 8-(N-piperidinyl)octanol, 10-(N-piperidinyl)decanol, 12-(N-piperidinyl)dodecanol, 2-(N-pyrrolidinyl)ethanol, 3-(N-pyrrolidinyl)propanol, 4-(N-pyrrolidinyl)butanol, 5-(N-pyrrolidinyl)pentanol, 6-(N-pyrrolidinyl)hexanol, 8-(N-pyrrolidinyl)octanol, 10-(N-pyrrolidinyl)decanol, 12-(N-pyrrolidinyl)dodecanol, 2-(N-morpholino)ethanol, 3-(N-morpholino)propanol, 4-(N-morpholino)butanol, 5-(N-morpholino)pentanol, 6-(N-morpholino)hexanol, 8-(N-morpholino)octanol, 10-(N-morpholino)decanol, 12-(N-morpholino)dodecanol, 2-(N'-methyl-N-piperazinyl)-ethanol, 3-(N'-methyl-N-piperazinyl)propanol, 4-(N'-methyl-N-piperazinyl)butanol, 5-(N'-methyl-N-piperazinyl)pentanol, 6-(N'-methyl-N-piperazinyl)hexanol, 8-(N'-methyl-N-piperazinyl)octanol, 10-(N'-methyl-N-piperazinyl)decanol, 12-(N'-methyl-N-piperazinyl)dodecanol, 2-(N'-ethyl-N-piperazinyl)ethanol, 3-(N'-ethyl-N-piperazinyl)propanol, 4-(N'-ethyl-N-piperazinyl)butanol, 5-(N'-ethyl-N-piperazinyl)pentanol, 6-(N'-ethyl-N-piperazinyl)hexanol, 8-(N'-ethyl-N-piperazinyl)octanol, 10-(N'-ethyl-N-piperazinyl)decanol, 12-(N'-ethyl-N-piperazinyl)dodecanol, 2-(N'-isopropyl-N-piperazinyl)-ethanol, 3-(N'-*iso*propyl-N-piperazinyl)propanol, 4-(N'-*iso*propyl-N-piperazinyl)butanol, 5-(N'-isopropyl-N-piperazinyl)pentanol, 6-(N'-*iso*propyl-N-piperazinyl)hexanol, 8-(N'-isopropyl-N-piperazinyl)octanol, 10-(N'-*iso*propyl-N-piperazinyl)decanol, 12-(N'-*iso*propyl-N-piperazinyl)dodecanol, 3-oxabutanol, 3-oxapentanol, 2,2-dimethyl-4-oxapentanol, 3,6-dioxaheptanol, 3,6-dioxaoctanol, 3,6,9-trioxadecanol, 3,6,9-trioxaundecanol, 4-oxapentanol, 4-oxahexanol, 4-oxaheptanol, 4,8-dioxanonanol, 4,8-dioxadecanol, 4,8-dioxaundecanol, 5-oxahexanol or 5,10-dioxaundecanol or ethoxylated and/or propoxylated alcohols and mixed ethoxylated/propoxylated alcohols, such as R⁵-(O-CH₂-CH₂)ₓ-OH or R⁵-(O-CH(CH₃)-CH₂)ₓ-OH or R⁵-(O-CH₂-CH(CH₃))ₓ-OH, where R⁵ is C₁ to C₂₀-alkyl and x is an integer from 1 to 20, or ethoxylated and/or propoxylated amino alcohols R³₂N(-CH₂CH₂-O)_{y}-H or R³₂N(-CH(CH₃)-CH₂-O)_{y}-H or R³₂N(-CH₂-CH(CH₃)-O-)_{y}-H, where y is an integer from 1 to 4, in the presence of a catalyst or catalyst mixture, wherein the lower alkanol R¹OH which is liberated in the transesterification and is selected from methanol, ethanol, n-propanol, isopropanol, n-butanol, isobutanol, 2-butanol and tert-butanol is separated off together with a part of the lower (meth)acrylate I and is fed, without an additional purification step, into a plant for production and/or working-up of the (meth)acrylate I and, from the discharge of the transesterification reaction,
either first the major amount of the (meth)acrylate I is separated off and is then separated by distillation from the catalyst used (catalyst removal),
or is first separated by distillation from the catalyst used (catalyst removal) and then the major amount of the (meth)acrylate I is separated off
and then components having a lower boiling point than the (meth)acrylate IV are substantially separated from the resulting mixture by distillation (low boiler removal) and then the (meth)acrylate IV is purified by distillation (distillate of purification),
the catalyst removal being carried out at from 125 to 160°C.

3. The process according to claim 2, wherein the alcohol R²OH is selected from dimethylaminoethanol, diethylaminoethanol, di-n-butylaminoethanol, 3-dimethylaminopropanol, 3-diethylaminopropanol and 3-di-n-butylaminopropanol.

4. The process according to any of the preceding claims, wherein the removal of the (meth)acrylate I is carried out at from 120 to 160°C.

5. The process according to any of the preceding claims, wherein the boiling point of the alcohol R²OH is 20°C or more above that of the alcohol R¹OH.

6. The process according to any of the preceding claims, wherein the distillate removed from the column attached to the reactor or from the columns attached to the reactors
comprises 20-40% by weight of methanol in the case of methanolate as the alcohol component (R¹O-) in the (meth)acrylate to be transesterified and
30-65% by weight of ethanol in the case of ethanolate as the alcohol component (R¹O-) in the (meth)acrylate I to be transesterified.

7. The process according to any of the preceding claims, wherein the residue of the catalyst removal is recycled at least partly to the transesterification.

8. The process according to any of the preceding claims, wherein the residue of the catalyst removal is subjected, separately or together with the residue of the distillative purification, at least partly to a thermal and/or catalytic treatment.

9. The process according to claim 7 or 8, wherein a distillate removed from the thermal and/or catalytic treatment is recycled to the low boiler removal and/or the transesterification.

10. The process according to any of claims 7 to 9, wherein the bottom discharge of the thermal and/or catalytic treatment is reacted with a high-boiling hydroxyl-comprising substance and the low boilers distilled off thereby are recycled to the transesterification.

11. The process according to any of the preceding claims, wherein at least a part of the discharge of the transesterification is recycled from an insulated buffer container to the transesterification.

12. The process according to claim 11, wherein the insulated buffer container is heated.

13. The process according to claim 11 or 12, wherein the insulated buffer container is connected to the last reactor of the transesterification and/or to the column attached to said reactor.

14. The process according to any of the preceding claims, wherein the catalyst used is a titanium alcoholate Ti(OR²)₄·

15. The process according to claim 14, wherein the titanium alcoholate Ti(OR²)₄ is obtained from a lower titanium alcoholate Ti(OR⁴)₄, where R⁴ is C₁-C₈-alkyl, by transesterification with a higher alcohol R²OH, where R² has the above meaning and R²OH and R⁴OH fulfill the condition b.p. (R²OH) ≥ b.p. (R⁴OH) + 20°C with regard to their boiling points b.p.

16. The process according to claim 15, wherein the alkanol R⁴OH formed in the transesterification is removed from the transesterification.

17. The process according to claim 15 or 16, wherein the reaction mixture obtained comprises less than 400 ppm of R⁴OH.

18. The process according to any of the preceding claims, which is carried out at least partly in the presence of at least one of the following stabilizers: phenothiazine, 4-methyl-2,6-tert-butylphenol, hydroquinone monomethyl ether, 4-hydroxy-2,2,6,6-tetramethylpiperidin-N-oxyl and 4-oxo-2,2,6,6-tetramethylpiperidin-N-oxyl.

19. The process according to claim 3, wherein the dialkylaminoethanol used has a vinyloxyethanol content of not more than 100 ppm.

20. The process according to claim 3, wherein the dialkylaminoethanol used has an ethylene glycol content of not more than 100 ppm.

21. The process according to any of the preceding claims, wherein R² is 2-(dimethylamino)ethyl, 2-(di-n-butylamino)ethyl or 2-(diethylamino)ethyl and R¹ is methyl or ethyl.

## Revendications

1. Procédé de préparation d'esters d'acide (méth)acrylique IV par transestérification d'un ester d'acide (méth)acrylique I avec un alcool R²OH, qui présente au moins un atome de carbone de plus que le composant alcool (R¹O-) dans l'ester d'acide (méth)acrylique I à transestérifier, en présence d'un catalyseur ou mélange de catalyseurs, **caractérisé en ce que** l'alcanol inférieur R¹OH libéré lors de la transestérification est séparé conjointement avec une partie de l'ester d'acide (méth)acrylique inférieur I et est, sans étape additionnelle de purification, alimenté dans une installation de préparation et/ou de traitement de l'ester d'acide (méth)acrylique I et, à partir de l'effluent réactionnel de la transestérification,
soit on sépare tout d'abord la quantité principale de l'ester d'acide (méth)acrylique I et on l'isole ensuite du catalyseur utilisé par distillation (séparation de catalyseur),
soit on l'isole tout d'abord du catalyseur utilisé par distillation (séparation de catalyseur) et ensuite on sépare la quantité principale de l'ester d'acide (méth)acrylique I,
et ensuite on isole sensiblement, par distillation, du mélange obtenu des composants à point d'ébullition plus bas que l'ester d'acide (méth)acrylique IV (séparation des substances à bas point d'ébullition) et ensuite on distille à l'état pur l'ester d'acide (méth)acrylique IV (distillation à l'état pur),
la séparation de catalyseur étant effectuée à des températures de 125 à 160°C.

2. Procédé de préparation d'esters d'acide (méth)acrylique IV par transestérification d'un ester d'acide (méth)acrylique I avec un alcool R²OH qui est choisi parmi du n-propanol, de l'iso-propanol, de l'alcool allylique, du n-butanol, du 1-méthyl-propanol, du 2-méthyl-propanol, du tert-butanol, du n-pentanol, du 1-méthyl-butanol, du 2-méthyl-butanol, du 3-méthyl-butanol, du 2,2-diméthylpropanol, du n-hexanol, du 1-méthyl-pentanol, du 2-méthyl-pentanol, du 3-méthyl-pentanol, du 4-méthyl-pentanol, du 1,1-diméthyl-butanol, du 2,2-diméthyl-butanol, du 3,3-diméthyl-butanol, du 1,2-diméthyl-butanol, du n-heptanol, du 1-méthyl-hexanol, du 2-méthyl-hexanol, du 3-méthyl-hexanol, du 4-méthyl-hexanol, du 1,2-diméthyl-pentanol, du 1,3-diméthyl-pentanol, du 1,1-diméthyl-pentanol, du 1,1,2,2-tétraméthylpropanol, de l'alcool benzylique, du n-octanol, du 2-éthylhexanol, du n-nonanol, du 1-méthyl-octanol, du 2-méthyl-octanol, du n-décanol, du n-undécanol, du 1-méthyl-décanol, du 2-méthyl-décanol, du n-dodécanol, du 2,4-diéthyl-octanol, du cyclopentanol, du cyclohexanol, du 4-tert-butylcyclohexanol, du cycloheptanol, du cyclododécanol, du 2-(diméthylamino)-éthanol, du 3-(diméthylamino)-propanol, du 4-(diméthylamino)-butanol, du 5-(diméthylamino)-pentanol, du 6-(diméthylamino)-hexanol, du 8-(diméthylamino)-octanol, du 10-(diméthylamino)-décanol, du 12-(diméthylamino)-dodécanol, du 2-(diéthylamino)-éthanol, du 3-(diéthylamino)-propanol, du 4-(diéthylamino)-butanol, du 5-(diéthylamino)-pentanol, du 6-(diéthylamino)-hexanol, du 8-(diéthylamino)-octanol, du 10-(diéthylamino)-décanol, du 12-(diéthylamino)-dodécanol, du 2-(di-(iso-propyl)-amino)-éthanol, du 3-(di-(iso-propyl)-amino)-propanol, du 4-(di-(iso-propyl)-amino)-butanol, du 5-(di-(iso-propyl)-amino)-pentanol, du 6-(di-(iso-propyl)-amino)-hexanol, du 8-(di-(iso-propyl)-amino)-octanol, du 10-(di-(iso-propyl)-amino)-décanol, du 12-(di-(iso-propyl)-amino)-dodécanol, du 2-(dibutylamino)-éthanol, du 3-(dibutylamino)-propanol, du 4-(dibutylamino)-butanol, du 5-(dibutylamino)-pentanol, du 6-(dibutylamino)-hexanol, du 8-(dibutylamino)-octanol, du 10-(dibutylamino)-décanol, du 12-(dibutylamino)-dodécanol, du 2-(dihexylamino)-éthanol, du 3-(dihexylamino)-propanol, du 4-(dihexylamino)-butanol, du 5-(dihexylamino)-pentanol, du 6-(dihexylamino)-hexanol, du 8-(dihexylamino)-octanol, du 10-(dihexylamino)-décanol, du 12-(dihexylamino)-dodécanol, du 2-(méthyl-éthyl-amino)-éthanol, du 2-(méthylpropyl-amino)-éthanol, du 2-(méthyl-iso-propylamino)-éthanol, du 2-(méthyl-butyl-amino)-éthanol, du 2-(méthyl-hexyl-amino)-éthanol, du 2-(méthyloctyl-amino)-éthanol, du 2-(éthyl-propyl-amino)-éthanol, du 2-(éthyl-iso-propyl-amino)-éthanol, du 2-(éthyl-butyl-amino)-éthanol, du 2-(éthyl-hexylamino)-éthanol, du 2-(éthyl-octyl-amino)-éthanol, du 3-(méthyl-éthyl-amino)-propanol, du 3-(méthylpropyl-amino)-propanol, du 3-(méthyl-iso-propylamino)-propanol, du 3-(méthyl-butyl-amino)-propanol, du 3-(méthyl-hexyl-amino)-propanol, du 3-(méthyl-octyl-amino)-propanol, du 3-(éthylpropyl-amino)-propanol, du 3-(éthyl-iso-propylamino)-propanol, du 3-(éthyl-butyl-amino)-propanol, du 3-(éthyl-hexyl-amino)-propanol, du 3-(éthyl-octyl-amino)-propanol, du 4-(méthyl-éthylamino)-butanol, du 4-(méthyl-propyl-amino)-butanol, du 4-(méthyl-iso-propyl-amino)-butanol, du 4-(méthyl-butyl-amino)-butanol, du 4-(méthylhexyl-amino)-butanol, du 4-(méthyl-octyl-amino)-butanol, du 4-(éthyl-propyl-amino)-butanol, du 4-(éthyl-iso-propyl-amino)-butanol, du 4-(éthylbutyl-amino)-butanol, du 4-(éthyl-hexyl-amino)-butanol, du 4-(éthyl-octyl-amino)-butanol, du 2-(N-pipéridinyl)-éthanol, du 3-(N-pipéridinyl)-propanol, du 4-(N-pipéridinyl)-butanol, du 5-(N-pipéridinyl)-pentanol, du 6-(N-pipéridinyl)-hexanol, du 8-(N-pipéridinyl)-octanol, du 10-(N-pipéridinyl)-décanol, du 12-(N-pipéridinyl)-dodécanol, du 2-(N-pyrrolidinyl)-éthanol, du 3-(N-pyrrolidinyl)-propanol, du 4-(N-pyrrolidinyl)-butanol, du 5-(N-pyrrolidinyl)-pentanol, du 6-(N-pyrrolidinyl)-hexanol, du 8-(N-pyrrolidinyl)-octanol, du 10-(N-pyrrolidinyl)-décanol, du 12-(N-pyrrolidinyl)-dodécanol, du 2-(N-morpholino)-éthanol, du 3-(N-morpholino)-propanol, du 4-(N-morpholino)-butanol, du 5-(N-morpholino)-pentanol, du 6-(N-morpholino)-hexanol, du 8-(N-morpholino)-octanol, du 10-(N-morpholino)-décanol, du 12-(N-morpholino)-dodécanol, du 2-(N'-méthyl-N-pipérazinyl)-éthanol, du 3-(N'-méthyl-N-pipérazinyl)-propanol, du 4-(N'-méthyl-N-pipérazinyl)-butanol, du 5-(N'-méthyl-N-pipérazinyl)-pentanol, du 6-(N'-méthyl-N-pipérazinyl)-hexanol, du 8-(N'-méthyl-N-pipérazinyl)-octanol, du 10-(N'-méthyl-N-pipérazinyl)-décanol, du 12-(N'-méthyl-N-pipérazinyl)-dodécanol, du 2-(N'-éthyl-N-pipérazinyl)-éthanol, du 3-(N'-éthyl-N-pipérazinyl)-propanol, du 4-(N'-éthyl-N-pipérazinyl)-butanol, du 5-(N'-éthyl-N-pipérazinyl)-pentanol, du 6-(N'-éthyl-N-pipérazinyl)-hexanol, du 8-(N'-éthyl-N-pipérazinyl)-octanol, du 10-(N'-éthyl-N-pipérazinyl)-décanol, du 12-(N'-éthyl-N-pipérazinyl)-dodécanol, du 2-(N'-iso-propyl-N-pipérazinyl)-éthanol, du 3-(N'-iso-propyl-N-pipérazinyl)-propanol, du 4-(N'-iso-propyl-N-pipérazinyl)-butanol, du 5-(N'-iso-propyl-N-pipérazinyl)-pentanol, du 6-(N'-iso-propyl-N-pipérazinyl)-hexanol, du 8-(N'-iso-propyl-N-pipérazinyl)-octanol, du 10-(N'-iso-propyl-N-pipérazinyl)-décanol, du 12-(N'-iso-propyl-N-pipérazinyl)-dodécanol, du 3-oxa-butanol, du 3-oxapentanol, du 2,2-diméthyl-4-oxa-pentanol, du 3,6-dioxa-heptanol, du 3,6-dioxa-octanol, du 3,6,9-trioxa-décanol, du 3,6,9-trioxa-undécanol, du 4-oxa-pentanol, du 4-oxa-hexanol, du 4-oxa-heptanol, du 4,8-dioxa-nonanol, du 4,8-dioxa-décanol, du 4,8-dioxa-undécanol, du 5-oxa-hexanol ou du 5,10-dioxa-undécanol ou des alcools éthoxylés et/ou propoxylés ainsi que des alcools éthoxylés/propoxylés mixtes comme R⁵-(O-CH₂-CH₂)ₓ-OH ou R⁵-(O-CH(CH₃)-CH₂)ₓ-OH ou respectivement R⁵-(O-CH₂-CH(CH₃))ₓ-OH, où R⁵ représente un groupe alkyle en C₁-C₂₀ et x un nombre entier entre 1 et 20, ou des aminoalcools éthoxylés et/ou propoxylés R³₂N(-CH₂CH₂-O)_{y}-H
ou R³₂N(-CH(CH₃)-CH₂-O)_{y}-H ou respectivement R³₂N(-CH₂-CH(CH₃)-O-)_{y}-H, où y représente un nombre entier entre 1 et 4, en présence d'un catalyseur ou mélange de catalyseurs, **caractérisé en ce que** l'alcanol inférieur R¹OH, qui est libéré au cours de la transestérification et qui est choisi parmi du méthanol, de l'éthanol, du n-propanol, de l'iso-propanol, du n-butanol, de l'iso-butanol, du 2-butanol et du tert-butanol, est séparé conjointement avec une partie de l'ester d'acide (méth)acrylique inférieur I et est, sans étape de purification additionnelle, alimenté dans une installation de préparation et/ou de traitement de l'ester d'acide (méth)acrylique I et, à partir de l'effluent réactionnel de la transestérification,
soit on sépare tout d'abord la quantité principale de l'ester d'acide (méth)acrylique I et on l'isole ensuite par distillation du catalyseur utilisé (séparation de catalyseur),
soit on sépare tout d'abord le catalyseur utilisé par distillation (séparation de catalyseur) et on isole ensuite la quantité principale de l'ester d'acide (méth)acrylique I,
et ensuite on isole sensiblement, par distillation, du mélange obtenu des composants à point d'ébullition plus bas que l'ester d'acide (méth)acrylique IV (séparation des substances à bas point d'ébullition) et ensuite on distille à l'état pur l'ester d'acide (méth)acrylique IV (distillation à l'état pur),
la séparation de catalyseur étant effectuée à des températures de 125 à 160°C.

3. Procédé suivant la revendication 2, **caractérisé en ce que** l'alcool R²OH est choisi parmi du diméthylaminoéthanol, du diéthylaminoéthanol, du di-n-butylaminoéthanol, du 3-diméthylaminopropanol, du 3-diéthylaminopropanol et du 3-di-n-butylaminopropanol.

4. Procédé suivant l'une des revendications précédentes, **caractérisé en ce qu'**on effectue la séparation de l'ester d'acide (méth)acrylique I à des températures de 120 à 160°C.

5. Procédé suivant l'une des revendications précédentes, **caractérisé en ce que** le point d'ébullition de l'alcool R²OH est situé à 20°C ou plus au-dessus de celui de l'alcool R¹OH.

6. Procédé suivant l'une des revendications précédentes, **caractérisé en ce que** le distillat prélevé de la colonne montée sur le réacteur/des colonnes montées sur les réacteurs contient
dans le cas de méthanolate comme composant alcool (R¹O-) dans l'ester d'acide (méth)acrylique I à transestérifier, 20 à 40 % en poids de méthanol, et
dans le cas d'éthanolate comme composant alcool (R¹O-) dans l'ester d'acide (méth)acrylique I à transestérifier, 30 à 65 % en poids d'éthanol.

7. Procédé suivant l'une des revendications précédentes, **caractérisé en ce que** le résidu de la séparation de catalyseur est au moins partiellement recyclé dans la transestérification.

8. Procédé suivant l'une des revendications précédentes, **caractérisé en ce que** le résidu de la séparation de catalyseur est, isolément ou conjointement avec le résidu de la distillation à l'état pur, soumis au moins partiellement à un traitement thermique et/ou catalytique.

9. Procédé suivant la revendication 7 ou 8, **caractérisé en ce qu'**un distillat prélevé du traitement thermique et/ou catalytique est recyclé dans la séparation de substances à bas point d'ébullition et/ou la transestérification.

10. Procédé suivant l'une des revendications 7 à 9, **caractérisé en ce que** l'effluent de fond de cuve du traitement thermique et/ou catalytique est amené à réagir avec une substance contenant des groupes hydroxyle, à point d'ébullition élevé, et les substances à bas point d'ébullition séparées par distillation sont recyclées dans la transestérification.

11. Procédé suivant l'une des revendications précédentes, **caractérisé en ce qu'**au moins une partie de l'effluent de la transestérification est recyclée à partir d'un récipient intermédiaire isolé à nouveau dans la transestérification.

12. Procédé suivant la revendication 11, **caractérisé en ce que** le récipient intermédiaire isolé est chauffé.

13. Procédé suivant la revendication 11 ou 12, **caractérisé en ce que** le récipient intermédiaire isolé est en communication avec le dernier réacteur de la transestérification et/ou la colonne montée sur celui-ci.

14. Procédé suivant l'une des revendications précédentes, **caractérisé en ce que**, comme catalyseur, on met en oeuvre un alcoolate de titane Ti(OR²)₄.

15. Procédé suivant la revendication 14, **caractérisé en ce que** l'alcoolate de titane Ti(OR²)₄ est obtenu à partir d'un alcoolate de titane inférieur Ti(OR⁴)₄, où R⁴ représente un groupe alkyle en C₁-C₈, par réaction avec un alcool supérieur R²OH, où R² a la signification donnée ci-dessus, et R²OH et R⁴OH remplissent en ce qui concerne leurs points d'ébullition P.E. la condition P.E. (R²OH) ≥ P.E. (R⁴OH) + 20°C.

16. Procédé suivant la revendication 15, **caractérisé en ce que** l'alcanol R⁴OH formé au cours de la réaction est éliminé de la réaction.

17. Procédé suivant la revendication 15 ou 16, **caractérisé en ce que** le mélange réactionnel obtenu contient moins de 400 ppm de R⁴OH.

18. Procédé suivant l'une des revendications précédentes, **caractérisé en ce qu'**il est effectué au moins partiellement en présence d'au moins un des stabilisants suivants : phénothiazine, 4-méthyl-2,6-tert-butylphénol, éther monométhylique d'hydroquinone, 4-hydroxy-2,2,6,6-tétraméthylpipéridin-N-oxyle, 4-oxo-2,2,6,6-tétraméthylpipéridin-N-oxyle.

19. Procédé suivant la revendication 3, **caractérisé en ce que** le dialkylaminoéthanol mis en oeuvre présente une teneur en vinyloxyéthanol qui n'est pas supérieure à 100 ppm.

20. Procédé suivant la revendication 3, **caractérisé en ce que** le dialkylaminoéthanol mis en oeuvre présente une teneur en éthylèneglycol qui n'est pas supérieure à 100 ppm.

21. Procédé suivant l'une des revendications précédentes, **caractérisé en ce que** R² est un groupe 2-(diméthylamino)-éthyle, 2-(di-n-butylamino)-éthyle ou 2-(diéthylamino)-éthyle et R¹ est un groupe méthyle ou éthyle.
